# EUROPÄISCHE PATENTSCHRIFT

(11) **EP 1 414 506 B1**
(45) Veröffentlichungstag und Bekanntmachung des Hinweises auf die Patenterteilung: **21.03.2007**
(21) Anmeldenummer: 02745037.8
(22) Anmeldetag: 22.07.2002
(51) Int. Cl.: A61M 5/00, A61M 5/315

(54) **Reservoirmodul mit Kolbenstange**
Reservoir module with a piston rod
Module de reservoir muni d'une tige de piston

(30) Priorität: 30.07.2001 DE 20112501 U; 21.12.2001 DE 10163327
(43) Veröffentlichungstag der Anmeldung: 06.05.2004
(73) Patentinhaber: TecPharma Licensing AG, 3401 Burgdorf (CH)
(72) Erfinder: KIRCHHOFER, Fritz, CH-3454 Sumiswald (CH); GRAF, Ronny, CH-3400 Burgdorf (CH)
(74) Vertreter: Schwabe - Sandmair - Marx
(86) Internationale Anmeldenummer: PCT/CH2002/000411
(87) Internationale Veröffentlichungsnummer: WO 2003/011372

(56) Entgegenhaltungen:
- EP-A- 0 295 075
- EP-A- 1 095 668
- WO-A-97/17095
- DE-A- 4 425 763

## Beschreibung

Die Erfindung betrifft ein Reservoirmodul, das eine Kolbenstange lagert, und ein Produktabgabegerät mit solch einem Reservoirmodul. Das Produktabgabegerät ist in bevorzugter Ausführungsform ein Verabreichungsgerät, beispielsweise ein Injektionsgerät oder Inhalationsgerät, für medizinische, therapeutische, diagnostische, pharmazeutische oder kosmetische Anwendungen. Bevorzugte Beispiele für Injektionsgeräte sind Injektionspens, insbesondere semidisposable Pens.

Die WO 97/17095 beschreibt ein Injektionsgerät, das aus einem Dosier- und Betätigungsmodul und einem Reservoirmodul besteht, die lösbar miteinander verbunden sind. Das Reservoirmodul ist als ein Einwegmodul konzipiert, während das Dosier- und Betätigungsmodul nach einem Verbrauch des Reservoirmoduls für eine Wiederverwendung mit einem neuen Reservoirmodul bestimmt ist. Das Reservoirmodul enthält ein Reservoir für ein zu injizierendes Produkt und lagert eine Kolbenstange, die für eine Produktausschüttung auf einen in dem Reservoir aufgenommenen Kolben wirkt. Die Kolbenstange weist ein Außengewinde auf, das mit einem Innengewinde eines Dosiseinstellglieds in einem Gewindeeingriff steht. Die Kolbenstange ist linear geführt, so dass bei einer Drehbewegung des Dosiseinstellglieds die Kolbenstange in Richtung auf den Kolben zu bewegt und dadurch ein lichter Abstand zwischen einem vorderen Ende der Kolbenstange und dem Kolben verändert wird. Das Reservoirmodul lagert auch das Dosiseinstellglied und umfasst somit die Kolbenstange und das Dosiseinstellglied, die nach der Entleerung des Reservoirs als ein einziges Modul zusammen mit dem Reservoir entsorgt werden.

Ein Vorteil des Konzepts des semidisposablen Injektionsgeräts ist, dass an der Dosierung und Ausschüttung beteiligte Teile des Injektionsgeräts nur für die Ausschüttung eines einzigen Reservoirinhalts ausgelegt werden müssen. Dies reduziert den Preis dieser Teile. Da im Falle der wiederholten Verwendung solche Teile von einem Verwender stets wieder in eine Ausgangsstellung zurückgeführt werden müssten, wären sie ferner einem nicht zu unterschätzenden Beschädigungsrisiko ausgesetzt. Die Sicherheit der korrekten Dosisauswahl und Dosisausschüttung muss daher bei semidisposablen Injektionsgeräten nicht geringer sein als bei vollständig wieder verwendbaren Geräten. Darüber hinaus ist der Austausch eines kompletten Reservoirmoduls einfacher als der Austausch nur eines Reservoirs.

Das Dosiseinstellglied des bekannten Geräts wird von einer Druckfeder, die von einem Gehäuse des Reservoirmoduls abgestützt wird, in eine hintere Stellung gedrückt, in der die Dosierung vorgenommen wird. Durch die Dosierung wird die Kolbenstange relativ zu dem Dosiseinstellglied und dem Gehäuse des Reservoirmoduls auf den Kolben zu vorbewegt. Die Produktausschüttung wird mittels einer Dosier- und Betätigungsvorrichtung bewirkt, die in einem Gehäuse des Dosier-und Betätigungsmoduls gelagert ist, und gegen eine rückwärtige Stirnfläche des Dosiseinstellglieds drückt. Die Dosier- und Betätigungsvorrichtung drückt das Dosiseinstellglied und damit zusammen aufgrund des Gewindeeingriffs auch die Kolbenstange in die Vorschubrichtung.

Aus der DE 44 25 763 A1 ist eine Vorrichtung zum Vorschub eines Kolbenverschiebeorgans bekannt, das zum Vorschub eines Kolbens in einem Zylinder bestimmt ist. Eine Sicherung gegen ein ungewolltes Verschieben des Vorschuborgans ist vorgesehen, die mit einer Feder als Sperrorgan ausgerüstet ist. Ein zu verabreichendes Medikament ist in einer Kartusche vorgesehen und ein Kolben in der Kartusche wird über eine Zahnstange vorangetrieben, die über einen Druckknopf betätigt werden kann. Die Zahnstange wird über eine Vorschubhülse vorangetrieben, wobei durch die Zahnteilung der Zahnstange die einzelnen Vorschubschritte für den Kolben unabänderlich vorgegeben sind.

Es ist eine Aufgabe der Erfindung, unter Verwendung eines als Kolbenstangenlagerung dienenden Reservoirmoduls den Preis von Produktabgabegeräten, insbesondere von Injektionsgeräten weiter zu verringern, dabei jedoch die Sicherheit in Bezug auf eine korrekte Auswahl und Ausschüttung einer Produktdosis zu erhalten.

Die gemäß der Erfindung erzielbaren Vorteile beruhen auf einem Produktabgabegerät nach dem Anspruch 1 sowie einem Reservoirmodul gemäß Anspruch 15. Zweckmäßige Ausführungsformen der erfindungsgemäßen Gegenstände gehen aus den Unteransprüchen hervor.

Die Erfindung betrifft ein Produktabgabegerät, insbesondere ein Injektionsgerät, mit einem Reservoirmodul und einem Dosier- und Betätigungsmodul, die lösbar miteinander verbunden sind. Vorzugsweise wird das Produktabgabegerät bereits durch diese beiden Module alleine gebildet. Das Reservoirmodul umfasst einen vorderen Gehäuseabschnitt des Produktabgabegeräts, der ein Reservoir für ein auszuschüttendes Produkt, das vorzugsweise Fluid und besonders bevorzugt injizierbar ist, und eine erste Verbindungseinrichtung aufweist. In dem Reservoir ist ein Kolben verschiebbar aufgenommen, so dass durch Verschiebung des Kolbens in eine Vorschubrichtung auf einen Reservoirauslass zu Produkt aus dem Reservoir ausgeschüttet wird. Das Reservoirmodul umfasst ferner eine Kolbenstange, die von dem vorderen Gehäuseabschnitt gehalten wird. Schließlich kann das Reservoirmodul eine Injektionsnadel oder beispielsweise eine Düse für nadellose Injektionen aufweisen.

Das Reservoir kann von einem Behältnis gebildet werden, das von dem Gehäuse aufgenommen ist. So kann insbesondere eine Ampulle das Reservoir bilden. Das Reservoir kann grundsätzlich aber auch unmittelbar von dem Gehäuse selbst gebildet werden, d.h. ohne Zwischenschaltung eines Produktbehältnisses. Bei dem Produkt handelt es sich vorzugsweise um eine Flüssigkeit für eine medizinische, therapeutische, diagnostische, pharmazeutische oder kosmetische Anwendung. Das Produkt kann beispielsweise Insulin oder ein Wachstumshormon sein. Das Produktabgabegerät ist vorzugsweise ein Injektionsgerät und wird vorzugsweise in solchen Verwendungen eingesetzt, in denen ein Verwender sich das Produkt selbst verabreicht, wie es beispielsweise in der Diabetestherapie üblich ist. Die Verwendung im stationären und ambulanten Bereich durch geschultes Personal soll jedoch nicht ausgeschlossen sein. Das Produkt kann in anderen Anwendungen, in denen eine dosierte Produktabgabe erforderlich oder auch nur erwünscht ist, beispielsweise auch ein Leim sein.

Die Kolbenstange kann mit dem Kolben fest, d.h. ständig, verbunden sein, worunter auch eine einstückige Ausbildung von Kolben und Kolbenstange verstanden werden soll. In bevorzugter Ausführung sind der Kolben und die Kolbenstange jedoch als separate Bauteile ausgeführt, und es drückt zum Zwecke der Produktausschüttung die Kolbenstange mit einem vorderen Ende gegen eine Rückseite des Kolbens.

Das Dosier- und Betätigungsmodul umfasst einen hinteren Gehäuseabschnitt des Produktabgabegeräts, der eine zweite Verbindungseinrichtung aufweist, die mit der ersten Verbindungseinrichtung die lösbare Verbindung zwischen dem Reservoirmodul und dem Dosier- und Betätigungsmodul bildet. Der vordere Gehäuseabschnitt und der hintere Gehäuseabschnitt bilden vorzugsweise das gesamte Gehäuse des Geräts. Die erste und zweite Verbindungseinrichtung können beispielsweise eine Drehverbindung, insbesondere eine Schraubverbindung bilden. Bevorzugter werden die beiden Gehäuseabschnitte jedoch linear aufeinander aufgeschoben, wobei die erste Verbindungseinrichtung und die zweite Verbindungseinrichtung eine Linearführung bilden, die eine Relativdrehung der beiden Gehäuseabschnitte zueinander verhindert. Ferner bilden sie zusammen vorzugsweise eine Verriegelungseinrichtung, so dass die übereinander geschobenen Bereiche der Gehäuseabschnitte nicht einfach wieder auseinander gezogen werden können.

Das Dosier- und Betätigungsmodul umfasst ferner eine Dosier- und Antriebsvorrichtung, mit der relativ zu den verbundenen Gehäuseabschnitten eine Dosierbewegung zur Auswahl einer Produktdosis und eine Ausschüttbewegung zur Ausschüttung der Produktdosis ausführbar sind.

Falls die Verbindungseinrichtungen eine Verriegelungseinrichtung bilden, umfasst diese Verriegelungseinrichtung vorzugsweise eine Verriegelungssperre, die eine Verriegelung der beiden Gehäuseabschnitte aneinander nur in einer vorderen Endposition, des Antriebselements oder Dosier- und Antriebselements der Dosier-und Antriebsvorrichtung zulässt.

Schließlich umfasst das Produktabgabegerät ein Dosiseinstellglied, das von der Dosier- und Antriebsvorrichtung bei der Ausschüttbewegung in die Vorschubrichtung bewegt wird und das mit der Kolbenstange und wenigstens einem der Gehäuseabschnitte je in solch einem Eingriff steht, dass es nur gemeinsam mit der Kolbenstange in die Vorschubrichtung bewegbar ist und durch die Dosierbewegung der Dosier- und Antriebsvorrichtung relativ zu der Kolbenstange entgegen der Vorschubrichtung bewegt wird. Der Eingriff mit der Kolbenstange ist vorzugsweise ein Gewindeeingriff. Grundsätzlich kann der Eingriff jedoch auch anders gebildet sein, beispielsweise als Zahneingriff, der die Bewegung des Dosiseinstellglieds relativ zu der Kolbenstange entgegen der Vorschubrichtung zulässt, aber eine in die Vorschubrichtung gerichtete Bewegung des Dosiseinstellglieds relativ zu der Kolbenstange verhindert. Das Dosiseinstellglied kann zwar grundsätzlich ein Bestandteil des Dosier- und Betätigungsmoduls sein, vorzugsweise ist es jedoch ein Bestandteil des Reservoirmoduls, d.h. es ist vorzugsweise bereits an dem Reservoirmodul gehalten bevor das Reservoirmodul mit dem Dosier- und Betätigungsmodul verbunden wird.

Nach der Erfindung weisen der vordere Gehäuseabschnitt eine Blockiereinrichtung und die Kolbenstange eine Rückzugsperreinrichtung auf, die miteinander in einem Sperreingriff stehen, der die Bewegung der Kolbenstange relativ zu dem vorderen Gehäuseabschnitt in die Vorschubrichtung zulässt, aber entgegen der Vorschubrichtung verhindert. Als gewünschter Nebeneffekt kann bereits durch den Sperreingriff sichergestellt sein, dass die Kolbenstange bei der Auswahl der Produktdosis relativ zu dem vorderen Gehäuseabschnitt nicht in die Vorschubrichtung bewegt wird. Falls die Kolbenstange durch den Sperreingriff allein hierfür noch nicht ausreichend sicher festgelegt wird, umfasst die Blockiereinrichtung auch noch eine Bremseinrichtung, um durch Ausübung einer zusätzlichen Reibkraft, eine Bewegung der Kolbenstange in die Vorschubrichtung bei der Auswahl der Produktdosis zu verhindern.

Bei dem Produktabgabegerät der Erfindung wird im Vergleich zu dem Gerät der WO 97/17095 die Druckfeder eingespart, die bei dem bekannten Gerät dafür sorgt, dass die Kolbenstange und das Dosiseinstellglied bei dem Dosiervorgang ihre hinterste Stellung einnehmen. Die Einsparung von Teilen bedeutet gleichzeitig eine Kosteneinsparung. Der Spareffekt wird dadurch verstärkt, dass die Reduzierung der Anzahl der Teile auf der Seite des Reservoirmoduls zu Buche schlägt. Da das erfindungsgemäße Reservoirmodul vorzugsweise als Einwegmodul konzipiert ist, macht sich der Spareffekt bei jedem Austausch solch eines Reservoirmoduls erneut bemerkbar.

Falls das Dosiseinstellglied die Dosierbewegung der Dosier- und Antriebsvorrichtung mitmacht, wird vorzugsweise sichergestellt, dass bei der Herstellung der hierfür erforderlichen Kopplung zwischen der Dosier- und Antriebsvorrichtung und dem Dosiseinstellglied, die durch das Verbinden der beiden Module herbeigeführt wird, eine Dosierbewegung des Dosiseinstellglieds nicht stattfinden kann. Für diesen Zweck werden das Dosiseinstellglied und das mit dem Dosiseinstellglied zu koppelnde Dosierelement oder kombinierte Dosier-und Antriebselement der Dosier- und Antriebsvorrichtung bei dem Verbinden der Module in bezüglich der Dosierbewegung vorgegebenen Positionen zueinander gehalten. Falls die Dosierbewegung eine Drehbewegung um eine in Richtung der Ausschüttbewegung weisende Drehachse ist, werden das Dosiseinstellglied und das Dosierelement oder Dosier- und Antriebselement bei dem Verbinden der Module in vorgegebenen Drehwinkelpositionen zueinander gehalten. Werden das Dosiseinstellglied hierbei von dem vorderen Gehäuseabschnitt und das Dosierelement oder Dosier- und Antriebselement von dem hinteren Gehäuseabschnitt je in einer vorgegebenen Drehwinkelposition gehalten, so kann vorteilhafterweise die genannte Linearführung der Gehäuseabschnitte sicherstellen, dass eine Dosierbewegung des Dosiseinstellglieds nicht durch die Herstellung der Kopplung bewirkt wird. Falls die Gehäuseabschnitte bei dem Verbinden der Module relativ zueinander eine Drehbewegung ausführen, wird anderweitig eine Dosierbewegung des Dosiseinstellglieds verhindert.

Das Produktabgabegerät kann eine Sperre aufweisen, die sicherstellt, dass das Reservoirmodul nur in einer vordersten Position der Kolbenstange von dem Dosier- und Betätigungsmodul gelöst werden kann. Zur sicheren Verhinderung eines nochmaligen Gebrauchs des Reservoirmoduls ist es in diesem Fall nur erforderlich, dass die Kolbenstange in ihrer vordersten Position so gehalten wird, dass der Verwender sie nicht wieder in eine axiale Position überführen kann, in der nach einem Reservoiraustausch eine erneute Produktausschüttung mit der gleichen Kolbenstange möglich ist. Vorzugsweise wird die Kolbenstange jedoch durch den Sperreingriff zwischen der an ihr ausgebildeten Rückzugssperreinrichtung und der Blockiereinrichtung des vorderen Gehäuseabschnitts nach jeder einzelnen Ausschüttung einer Produktdosis so gehalten, dass eine Überführung in eine frühere axiale Position verhindert wird. Falls ferner sichergestellt ist, dass die Kolbenstange und das Reservoir nicht voneinander getrennt werden können, wird ein Reservoiraustausch vorteilhafterweise in allen axialen Positionen der Kolbenstange verhindert. Der Sperreingriff bildet somit in jeder axialen Position Stellung der Kolbenstange auch gleichzeitig einen Sicherheitseingriff.

In bevorzugten Ausführungsbeispielen wird die Rückzugssperreinrichtung von Sägezähnen gebildet, die von der Kolbenstange abragen und eine Zahnreihe mit vorzugsweise regelmäßiger Teilung bilden. Die Erfindung hat erkannt, dass das für die Dosierung an sich bekannte Prinzip der Zahnstange mit Vorteil bei einem als Einwegmodul konzipierten Reservoirmodul zum Einsatz gebracht werden kann, um die Kosten für den Reservoirmodul und infolgedessen in besonderem Maße auch für das Produktabgabesystem im Ganzen zu senken. Eine Sägezahnreihe mit in Vorschubrichtung sich verjüngenden Zähnen und rückwärtigen Sperrflächen liefert in jedem Sperreingriff als Nebenprodukt auch gleichzeitig den Sicherheitseingriff, der eine Rückführung der Kolbenstange verhindert.

Der Sicherheitseingriff zwischen der Blockiereinrichtung und der Kolbenstange, der eine Rückführung der Kolbenstange verhindert, ist nicht lösbar. Nicht lösbar bedeutet in diesem Zusammenhang, dass er von einem Verwender nicht zerstörungsfrei gelöst werden kann. Allerdings soll nicht von vornherein ausgeschlossen sein, dass das Reservoirmodul in einen geschlossenen Kreislauf wieder aufgearbeitet werden kann und der Sperreingriff in diesem Falle von beispielsweise dem Hersteller mittels Spezialwerkzeug gelöst werden kann. Es wird allerdings sichergestellt, dass ein Verwender, der das Produktabgabegerät beispielsweise in der Selbstverabreichung verwendet, beispielsweise in der Verabreichung von Insulin oder Wachstumshormonen, das Reservoir nicht austauschen oder wieder auffüllen kann.

Die Dosier- und Antriebsvorrichtung kann manuell, halb automatisch oder voll automatisch arbeiten. Im ersten Fall wird sowohl die rotatorische Dosierbewegung als auch die translatorische Ausschüttbewegung manuell ausgeführt. Im zweiten Fall wird entweder die Dosierbewegung oder die Ausschüttbewegung manuell ausgeführt, und die andere Bewegung wird motorisch oder mittels einer anderen Art der Kraftbeaufschlagung, beispielsweise mittels Federkraft bewirkt, wenn der Verwender die entsprechende Bewegung durch einen Betätigungshandgriff ausgelöst hat. Im dritten Fall, der vollautomatischen Dosier- und Antriebsvorrichtung werden die Dosierbewegung und die Ausschüttbewegung motorisch oder mittels einer anderen Kraft, beispielsweise Federkraft, bewirkt. Es wird in diesem Fall manuell lediglich die Dosis ausgewählt, beispielsweise mittels einer oder mehreren Tasten, und es wird die Ausschüttbewegung ebenfalls durch einen eigenen, entsprechenden Betätigungshandgriff des Verwenders ausgelöst. In den meisten Ausführungen ist das erfindungsgemäße Verabreichungsgerät mit einer manuellen Dosier- und Antriebsvorrichtung ausgerüstet, die dann als Dosier-und Betätigungsvorrichtung bezeichnet wird. Soweit daher von einer Dosier- und Betätigungsvorrichtung die Rede ist, wird damit die manuelle Ausführung bezeichnet. Soweit von einer Dosier- und Antriebsvorrichtung die Rede ist, soll hierdurch keine Beschränkung in Bezug auf manuell, halb automatisch oder voll automatisch vorgenommen werden, sondern es soll jede der Ausführungen umfasst sein. Der Begriff "Dosier- und Betätigungsmodul" wird jedoch im Zusammenhang mit sämtlichen Ausführungen der Dosier- und Antriebsvorrichtung verwendet.

Die Dosier- und Antriebsvorrichtung kann ein Dosierelement, das die Dosierbewegung ausführt, und separat ein Antriebselement aufweisen, das die Ausschüttbewegung ausführt. Vorzugsweise werden die Dosierbewegung und Ausschüttbewegung jedoch von dem gleichen Körper der Dosier- und Antriebsvorrichtung ausgeführt, der im Folgenden daher auch als Dosier- und Antriebselement oder als Dosier- und Betätigungselement bezeichnet wird.

Bei dem Produkt handelt es sich vorzugsweise um ein Fluid, besonders bevorzugt eine Flüssigkeit, für eine medizinische, therapeutische, diagnostische, pharmazeutische oder kosmetische Anwendung. Das Produkt kann beispielsweise Insulin, ein Wachstumshormon oder auch dünn- bis dickflüssige, breiförmige Nahrung sein. Das Verabreichungsgerät wird vorzugsweise in solchen Verwendungen eingesetzt, in denen ein Verwender sich das Produkt selbst verabreicht, wie es beispielsweise in der Diabetestherapie üblich ist. Die Verwendung im stationären und ambulanten Bereich durch geschultes Personal soll jedoch nicht ausgeschlossen sein.

Die Produktverabreichung kann bei einem Injektionsgerät mittels Injektionskanüle oder beispielsweise einer Düse für nadellose Injektionen erfolgen. Die Injektion oder Infusion kann insbesondere subkutan oder venös vorgenommen werden, oder auch intramuskulär. Im Falle der Verabreichung per Inhalation kann die ausgewählte Produktdosis aus dem Reservoir beispielsweise in eine Kammer des Inhalationsgeräts ausgeschüttet und mittels einer Zerstäubungseinrichtung für die Inhalation zerstäubt werden. Denkbar ist ferner eine orale Einnahme oder eine Verabreichung über die Speiseröhre, um nur einige Beispiele der Verabreichung zu nennen.

Besonders bevorzugt ist das Verabreichungsgerät semidisposable. In diesem Fall ist der vordere Gehäuseabschnitt Träger eines Reservoirmoduls, das nach Entleerung des Reservoirs entsorgt oder in einen Kreislauf zurückgeführt wird, und der hintere Gehäuseabschnitt ist Träger eines Dosier- und Betätigungsmoduls, das in Verbindung mit einem neuen Reservoirmodul wiederholt verwendbar ist. Da das Reservoirmodul als Verbrauchsmodul auch separat gehandelt wird, ist es auch separat Gegenstand der Erfindung. Auch das Dosier- und Betätigungsmodul kann separat Gegenstand der Erfindung sein. Ebenso bildet ein System aus einem Verabreichungsgerät und wenigstens einem Reservoirmodul, das das Reservoirmodul des Geräts nach Verbrauch ersetzen kann, einen Gegenstand der Erfindung. Das Konzept des zweigeteilten Verabreichungsgeräts in einen für die nur einmalige Verwendung vorgesehenen Teil und einen für die wiederholte Verwendung vorgesehenen Teil (semidisposable) ist vorteilhaft insbesondere für Injektionspens, aber desweiteren auch für beispielsweise Inhalationsgeräte oder Geräte für die orale Einnahme eines Produkts oder eine künstliche Ernährung.

In den Unteransprüchen werden weitere bevorzugte Ausgestaltungen der Erfindung beschrieben, wobei Merkmale die nur in Bezug auf das Verabreichungsgerät oder nur in Bezug auf ein Reservoirmodul oder ein Dosier-und Betätigungsmodul beansprucht sind, auch bevorzugte Merkmale in Bezug auf den jeweils anderen Anspruchsgegenstand sind.

Ausführungsbeispiele der Erfindung werden nachfolgend anhand von Figuren beschrieben. An den Ausführungsbeispielen offenbar werdende Merkmale bilden je einzeln und in jeder Merkmalskombination die Gegenstände der Ansprüche vorteilhaft weiter. Auch Merkmale, die nur an dem einen Beispiel offenbart sind, bilden das jeweils andere Beispiel weiter oder zeigen eine Alternative auf, soweit nichts Gegenteiliges offenbart wird oder nur der Fall sein kann. Es zeigen:
- Figur 1: zwei Teile eines Reservoirmoduls nach einem ersten Ausführungsbeispiel,
- Figur 2: das aus den zwei Teilen der Figur 1 erhaltene Reservoirmodul,
- Figur 3: ein das Reservoirmodul der Figur 2 umfassendes Injektionsgerät nach dem ersten Ausführungsbeispiel in einem Längsschnitt,
- Figur 4: einen Teil des Injektionsgeräts der Figur 3,
- Figur 5: einen Mechanikhalter des Reservoirmoduls in einem Längsschnitt und zwei Ansichten,
- Figur 6: eine von dem Mechanikhalter gelagerte Blockiereinrichtung für eine Kolbenstange,
- Figur 7: eine Kolbenstange in einem Längsschnitt und einer Stirnansicht,
- Figur 8: eine Verriegelungssperre in einem Längsschnitt, einer Ansicht und einer Draufsicht,
- Figur 9: ein zweites Ausführungsbeispiel eines Injektionsgeräts,
- Figur 10: den Querschnitt A-A der Figur 9,
- Figur 11: den Querschnitt B-B der Figur 9,
- Figur 12: den Querschnitt C-C der Figur 9,
- Figur 13: den Querschnitt D-D der Figur 9,
- Figur 14: den Mechanikhalter des zweiten Ausführungsbeispiels in einer perspektivischen Darstellung,
- Figur 15: den Mechanikhalter der Figur 14 in einer Ansicht,
- Figur 16: den Querschnitt A-A der Figur 15,
- Figur 17: das Dosiseinstellglied des zweiten Ausführungsbeispiels in einer perspektivischen Darstellung,
- Figur 18: das Dosiseinstellglied der Figur 17 in einem Längsschnitt,
- Figur 19: das Dosiseinstellglied der Figur 17 in einer Ansicht,
- Figur 20: das Dosiseinstellglied der Figur 17 in einer Draufsicht,
- Figur 21: einen Teil des Injektionsgeräts nach Figur 3 und
- Figur 22: einen Teil des Injektionsgeräts nach Figur 9.

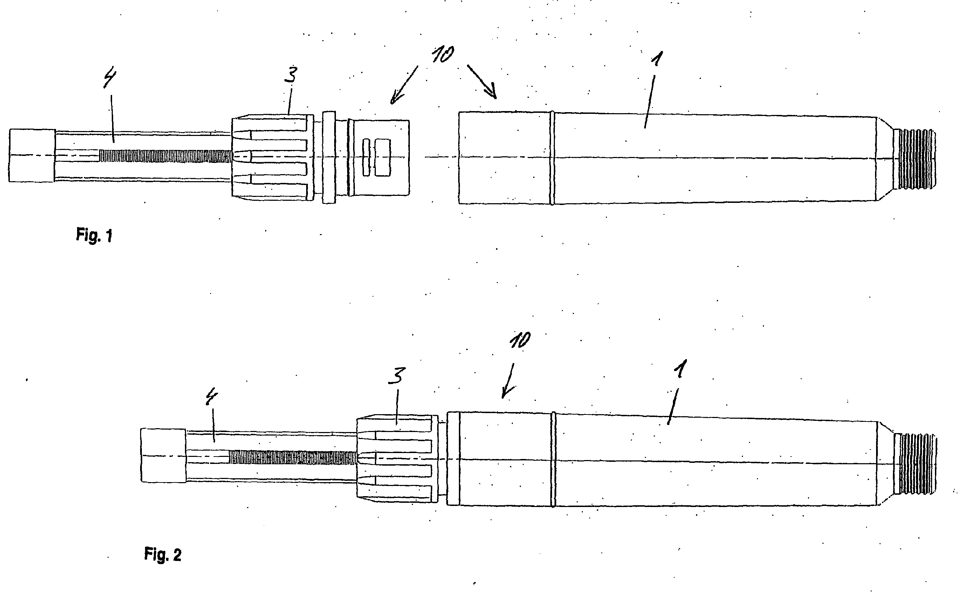

Figur 1 zeigt in einer Ansicht ein Reservoirteil 1 und einen Mechanikhalter 3, die miteinander verbunden werden, um das in Figur 2 dargestellte Reservoirmodul 10 zu bilden.

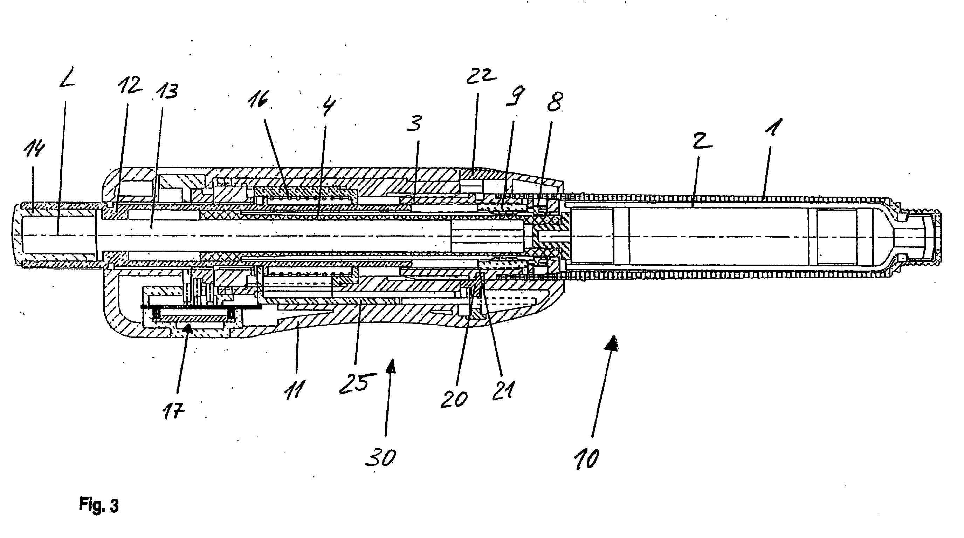

In den Figuren 1 und 2 ist ferner eine Kolbenstange 4 erkennbar, die an einem von dem Reservoirteil 1 abgewandten Ende des Mechanikhalters 3 in den Mechanikhalter 3 hineinragt und von dem Mechanikhalter 3 in eine in Längsachse L der Kolbenstange 4 weisende Vorschubrichtung auf ein von dem Mechanikhalter 3 abgewandtes, vorderes Ende des Reservoirteils 1 zu verschiebbar gelagert wird. Das Reservoirteil 1 ist im Wesentlichen ein im Querschnitt kreisförmiger Hohlzylinder, der an seinem vorderen Ende einen Verbindungsbereich für eine Verbindung mit einem Nadelhalter für eine Injektionsnadel aufweist. Das Reservoirteil 1 dient der Aufnahme eines Reservoirbehältnisses, das im Ausführungsbeispiel von einer Ampulle 2 gebildet wird, die in dem Längsschnitt der Figur 3 erkennbar ist. Ein Auslass an dem vorderen Ende der Ampulle 2 wird von einer Membran fluiddicht verschlossen. Bei der Befestigung des Nadelhalters an dem vorderen Ende des Reservoirteils 1 durchsticht ein rückwärtiger Teil der Injektionsnadel die Membran, so dass eine Fluidverbindung zwischen der Spitze der hohlen Injektionsnadel und dem Reservoir 2 hergestellt ist.

Figur 3 zeigt das Injektionsgerät in seiner Gesamtheit in einem Längsschnitt. In der Ampulle 2 ist ein Kolben in die Vorschubrichtung auf den am vorderen Ende der Ampulle 2 gebildeten Auslass zu verschiebbar aufgenommen. Durch die Verschiebung des Kolbens in Vorschubrichtung wird Produkt aus der Ampulle 2 verdrängt und durch den Auslass und die Injektionsnadel hindurch ausgeschüttet.

Den Vorschub des Kolbens bewirkt die Kolbenstange 4, die mit ihrem vorderen Ende gegen den Kolben drückt und dadurch bei ihrem eigenen Vorschub den Kolben in die Vorschubrichtung bewegt. Die Kolbenstange 4 wird von dem Mechanikhalter 3 so gehalten, dass sie nach Überwindung eines gewissen Widerstands in die Vorschubrichtung, nicht jedoch entgegen der Vorschubrichtung bewegt werden kann. Die Rückwärtsbewegung der Kolbenstange 4 entgegen der Vorschubrichtung wird von einer Blockiereinrichtung 8 verhindert. Die Blockiereinrichtung 8 wird von dem Mechanikhalter 3 axial fixiert, d.h. sie ist in dem Mechanikhalter 3 so gehalten, dass sie in und gegen die Vorschubrichtung nicht bewegbar ist. Allerdings wird sie von dem Mechanikhalter 3 um die Längsachse L drehbar gelagert. Die Blockiereinrichtung 8 erzeugt auch den Widerstand, der für die Vorwärtsbewegung überwunden werden muss.

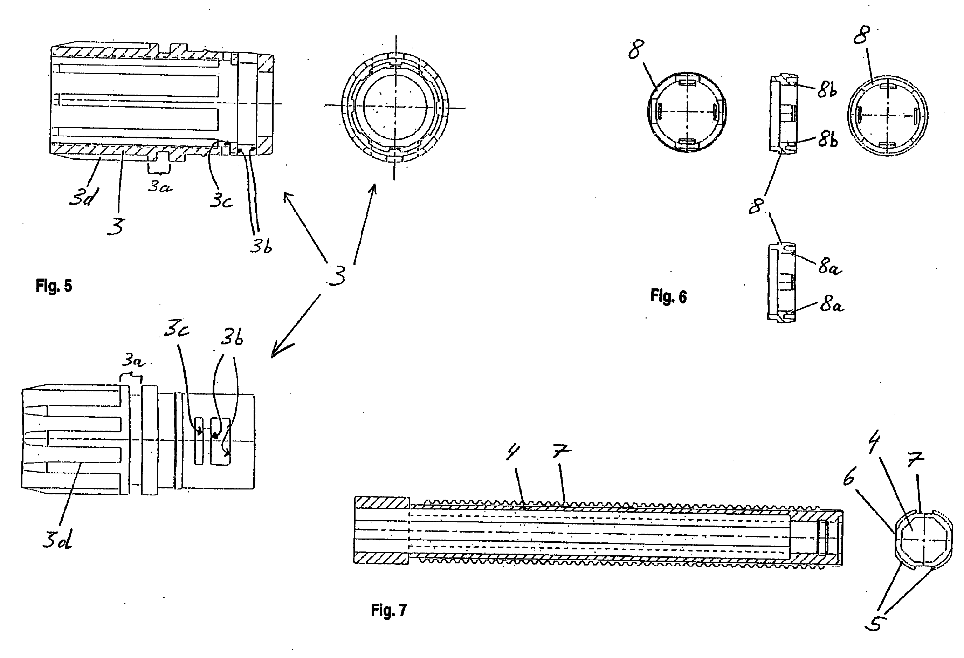

Die Blockiereinrichtung 8 ist in Figur 6 alleine dargestellt. Sie wird von einem einteiligen Ringelement gebildet, das an dem Mechanikhalter 3 zwischen zwei einander zugewandt beabstandeten Schultern 3b, die von einem Innenmantel des Mechanikhaltes 3 radial nach innen vorstehen, um die Längsachse L drehbar anliegt. Die Schultern 3b bilden eine Fixiereinrichtung für die axiale Fixierung der Blockiereinrichtung 8. Die Lagerung der Blockiereinrichtung 8 in dem Mechanikhalter 3 ist am besten aus der Darstellung des Mechanikhalters 3 in Figur 5 ersichtlich.

In dem Mechanikhalter 3 ist ferner ein Dosiseinstellglied 9 aufgenommen. Das Dosiseinstellglied 9 ist als Gewindemutter ausgebildet und steht mit einem Außengewinde der Kolbenstange 4 in einem Gewindeeingriff. Das Dosiseinstellglied 9 wird von dem Mechanikhalter 3 verdrehgesichert, aber in und gegen die Vorschubrichtung axial linear bewegbar geführt. Die Kolbenstange 4 und das Dosiseinstellglied 9 bilden einen Spindeltrieb, um die zu verabreichende Produktdosis auszuwählen.

Der Ampullenhalter 1 und der Mechanikhalter 3 sind verdreh- und verschiebegesichert miteinander verbunden und bilden zusammen das Reservoirmodul 10 des Injektionsgeräts, wobei dieses Reservoirmodul 10 die von dem Mechanikhalter 3 mittels der Blockiereinrichtung 8 gehaltene Kolbenstange 4 und das Dosiseinstellglied 9 umfasst. Der Ampullenhalter 1 und der Mechanikhalter 3 bilden zusammen einen vorderen Gehäuseabschnitt des Injektionsgeräts. Mit diesem vorderen Gehäuseabschnitt 1, 3 ist ein hinterer Gehäuseabschnitt 11 formschlüssig verbunden. Der hintere Gehäuseabschnitt 11 bildet den Träger eines Dosier- und Betätigungselements 12 und zusammen mit demselben sowie Teilen einer Verriegelungseinrichtung und weiteren Teilen ein Dosier- und Betätigungsmodul 30 des Injektionsgeräts.

Eine Dosier- und Betätigungsvorrichtung umfasst mit Ausnahme des Dosiseinstellglieds 9, der Kolbenstange 4 und der Blockiereinrichtung 8 die anderen Komponenten für die Auswahl der Produktdosis und die Betätigung des Injektionsgeräts. Insbesondere umfasst sie das Dosier- und Betätigungselement 12. Ferner umfasst die Dosier- und Betätigungsvorrichtung eine Zähl- und Anzeigeeinrichtung 17 zum Zählen und optischen Anzeigen der ausgewählten Produktdosis. Nicht zuletzt die Zähl- und Anzeigeeinrichtung 17 macht das Dosier-und Betätigungsmodul 30 zu einem hochwertigen und daher hochpreisigen Teil des Injektionsgeräts. Während das vergleichsweise preiswerte Reservoirmodul 10 als Einwegmodul konzipiert ist, ist das Dosier- und Betätigungsmodul 30 für die mehrmalige Verwendung mit immer wieder neuen Reservoirmodulen 10 bestimmt.

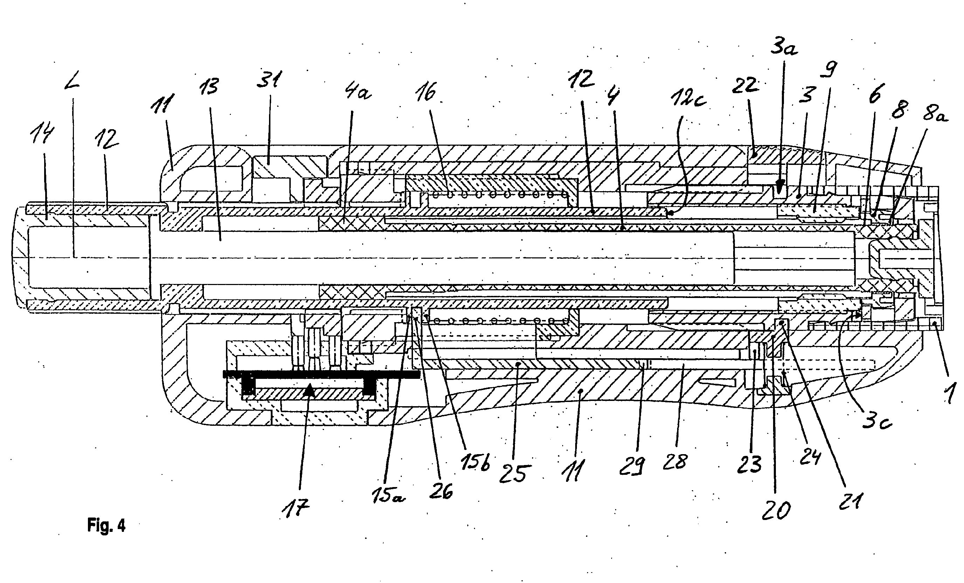

Für die Auswahl der Produktdosis, d.h. für die Dosierung, wird das Dosier- und Betätigungselement 12 um die Längsachse L drehbar und ferner in und gegen die Vorschubrichtung entlang der Längsachse L geradverschiebbar von dem hinteren Gehäuseabschnitt 11 gelagert. Das Dosier- und Betätigungselement 12 ist hohlzylindrisch und umgibt mit einem vorderen Abschnitt die Kolbenstange 4. Ein hinterer Abschnitt des Dosier- und Betätigungselements 12 ragt über ein hinteres Ende des Gehäuseabschnitts 11 hinaus. In das Dosier- und Betätigungselement 12 ist von hinten ein stangenförmiger Dosiermitnehmer 13 bis gegen eine radial nach innen vorragende Schulter des Dosier- und Betätigungselements 12 eingeschoben. Ferner ist an dem hinteren Ende ein Verschluss 14 bis gegen den Dosiermitnehmer 13 in das Dosier- und Betätigungselement 12 eingeschoben. Der Dosiermitnehmer 13 wird zwischen der radial vorragenden Schulter des Dosier-und Betätigungselements 12 und dem Verschluss 14 relativ zu dem Dosier- und Betätigungselement 12 axial fixiert. Der Dosiermitnehmer 13 ist mit dem Dosier-und Betätigungselement 12 außerdem verdrehgesichert verbunden. Der Dosiermitnehmer 13 ragt zum Zwecke der Dosierung von hinten in die hohle Kolbenstange 4 hinein. Die Kolbenstange 4 weist einen Verbindungabschnitt 4a auf (Fig. 4), der mit dem Dosiermitnehmer 13 in solch einem Eingriff ist, dass die Kolbenstande 4 und der Dosiermitnehmer 13 und mit diesem auch das Dosier-und Betätigungselement 12 relativ zueinander um die gemeinsame Längsachse L nicht verdrehbar, aber entlang der Längsachse L in und gegen die Vorschubrichtung relativ zueinander bewegbar sind. Hierfür ist der Verbindungsabschnitt 4a als Linearführung für den Dosiermitnehmer 13 ausgebildet.

Eine Rückstelleinrichtung 16 spannt das Dosier- und Betätigungselement 12 elastisch gegen die Vorschubrichtung in die in den Figuren 3 und 4 dargestellte Ausgangsstellung. In der Ausgangsstellung kann durch Drehung des Dosier- und Betätigungselements 12 um die Längsachse L die Dosierung vorgenommen werden. Anschließend kann aus der Ausgangsstellung heraus durch Axialverschiebung des Dosier- und Betätigungselements 12 die Ausschüttung der ausgewählten Produktdosis bewirkt werden. Die Rückstelleinrichtung 16 wird von einer als Druckfeder wirkenden Spiralfeder gebildet, die in einem Ringspalt um das Dosier- und Betätigungselement 12 aufgenommen ist und zwischen einer radial nach innen ragenden Schulter des Gehäuseabschnitts 11 und einer gegenüberliegend zugewandt radial nach außen ragenden Schulter des Dosier-und Betätigungselements 12 axial abgestützt ist.

Die Blockiereinrichtung 8 erfüllt eine Doppelfunktion. Zum einen stellt sie mit ihren Sperrelementen 8a sicher, dass die Kolbenstange 4 relativ zu dem Mechanikhalter 3 und damit insbesondere relativ zu dem in der Ampulle 2 aufgenommenen Kolben nicht entgegen der Vorschubrichtung zurückbewegt werden kann. In ihrer Doppelfunktion verhindert die Blockiereinrichtung 8 ferner als Bremse eine Vorwärtsbewegung der Kolbenstange 4 bei dem Dosiervorgang, bei dem das Dosiseinstellglied 9 entgegen der Vorschubrichtung axial auf das Dosier- und Betätigungselement 12 zu bewegt wird.

In der in den Figuren 3 und 4 dargestellten Ausgangsstellung vor einer Dosierung liegt das Dosiseinstellglied 9 in Vorschubrichtung auf Anschlag gegen einen von dem Mechanikhalter 3 gebildeten Ausschüttanschlag 3c (Figur 5). Die Kolbenstange 4 steht in einem permanenten Berührungskontakt mit dem Kolben. Zum Zwecke der Dosierung wird das Dosiseinstellglied 9 durch den Gewindeeingriff mit der Kolbenstange 4 und die Linearführung durch den Mechanikhalter 3 von dem Ausschüttanschlag 3c weg auf das Dosier- und Betätigungselement 12 zu bewegt. Hierdurch wird ein lichter Abstand zwischen einer rückwärtigen Anschlagfläche des Dosiseinstellglieds 9 und einer vorderen Anschlagfläche des Dosier- und Betätigungselements 12 verringert, andererseits jedoch der lichte Abstand zwischen einer vorderen Anschlagfläche des Dosiseinstellglieds 9 und dem Ausschüttanschlag 3c vergrößert. Der letztgenannte Abstand zwischen dem Ausschüttanschlag 3c und dem Dosiseinstellglied 9 ist die Weglänge, um die im Zuge der Ausschüttbewegung des Dosier- und Betätigungselements 12 das Dosiseinstellglied 9 und wegen des Gewindeeingriffs auch die Kolbenstange 4 in die Vorschubrichtung bewegt werden. Der Ausschüttanschlag 3c bildet einen vorderen Translationsanschlag. Bei der Ausschüttbewegung drückt die Kolbenstange 4 mit ihrem vorderen Ende, das von einem mit der Kolbenstange 4 in und gegen die Vorschubrichtung nicht bewegbar verbundenen Stempelkörper gebildet wird, gegen den Kolben und drückt den Kolben in die Vorschubrichtung auf den Auslass der Ampulle 2 zu vor. Die Längsachse L bildet die Rotations- und Translationsachse der Bewegungen, die zum Zwecke der Dosierung und Produktausschüttung ausgeführt werden.

Der Abstand, den das Dosiseinstellglied 9 und das Dosier- und Betätigungselement 12 vor dem Dosiervorgang zwischen sich aufweisen, wenn das Dosiseinstellglied 9 auf Anschlag gegen den Ausschüttanschlag 3c liegt, entspricht der maximalen Produktdosis, die ausgewählt und im Zuge einer Ausschüttung ausgeschüttet werden kann. Die Hubbewegung des Dosier- und Betätigungselements 12 ist bei jeder Ausschüttung gleich lang. Es wird durch die Dosierung lediglich der Abstand des Dosiseinstellglieds 9 von dem Ausschüttanschlag 3c und damit die im Zuge der Ausschüttung von dem Dosier-und Betätigungselement 12 und dem Dosiseinstellglied 9 gemeinsam zurücklegbare Weglänge eingestellt.

Die Bremsfunktion der Blockiereinrichtung 8 und der hierfür zwischen der Kolbenstange 4 und der Blockiereinrichtung 8 bestehende Bremseingriff werden aus der Zusammenschau der Figuren 6 und 7 deutlich. Zum einen weist die Blockiereinrichtung 8 für den Bremseingriff zwei Bremselemente 8b auf, die, wie bereits die Sperrelemente 8a, je von einem elastisch nachgebenden Schnapper gebildet werden. Die Blockiereinrichtung 8 wird im Ausführungsbeispiel von einem einzigen Ringelement gebildet, von dem an einer Stirnseite vier elastische Schnapper axial abragen. Die Schnapper sind gleichmäßig über den Umfang des Ringelements verteilt angeordnet. Zwei einander gegenüberliegende Schnapper bilden die Sperrelemente 8a und die zwei weiteren, einander ebenfalls gegenüberliegend angeordneten Schnapper bilden die Bremselemente 8b.

Die Kolbenstange 4 weist dementsprechend zwei am Außenmantel an gegenüberliegenden Seiten ausgebildete und in Längsrichtung der Kolbenstange 4 sich erstreckende Rückzugsperreinrichtungen 6 und zwei an ebenfalls einander gegenüberliegenden Seiten in Längsrichtung der Kolbenstange 4 sich erstreckende Vorschubbremseinrichtungen 7 auf. Das Gewinde der Kolbenstange 4 für den Gewindeeingriff mit dem Dosiseinstellglied 9 wird von vier verbleibenden Gewindeabschnitten 5 gebildet, die sich über nahezu die gesamte Länge der Kolbenstange 4 erstrecken. Die Rückzugsperreinrichtungen 6 und die Vorschubbremseinrichtungen 7 werden je von einer Zahnreihe gebildet. Während die Zähne der Rückzugsperreinrichtungen 6 jedoch als in Vorschubrichtung gepfeilte Sägezähne mit nach hinten weisenden, quer zur Vorschubrichtung sich erstreckenden Sperrflächen gebildet sind, weisen die beiden Zahnreihen, die die Vorschubbremseinrichtungen 7 bilden, nach vorne weisende Sperrflächen mit einer vergleichbaren Sperrwirkung nicht auf. Die Zähne der Vorschubbremseinrichtungen 7 weisen je ein im Vergleich zu den Rückzugssperreinrichtungen 6 weicheres Zahnprofil auf. Der Bremseingriff der Blockiereinrichtung 8 mit den Vorschubbremseinrichtungen 7 der Kolbenstange 4 soll eine Vorschubbewegung der Kolbenstange 4 nämlich nicht verhindern, sondern nur erschweren, um sicherzustellen, dass die Kolbenstange 4 nicht bei der Dosierung in Vorschubrichtung bewegt wird. Die Zähne der Vorschubbremseinrichtungen 7 sind an ihren Vorderseiten und die Bremselemente 8b sind an ihren Rückseiten, die die Vorderseiten der Zähne der Vorschubbremseinrichtung 7 berühren, so geformt, dass zur Überwindung des Bremseingriffs eine Schwellkraft überwunden werden muss, die bei der Dosierung nicht erreicht wird. Diese Schwellkraft ist größer als die Kraft, die erforderlich ist, um die Zähne der Rückzugssperreinrichtungen 6 über die Sperrelemente 8a in Vorschubrichtung zu bewegen. Vorzugsweise ist die Schwellkraft wenigstens doppelt so groß wie die anfängliche Reibkraft zwischen den Rückzugssperreinrichtungen 6 und den Sperrelementen 8a. Die Reibkraft zwischen letzteren vergrößert sich auch erst im Verlaufe der Vorschubbewegung allmählich zwischen zwei aufeinanderfolgenden Sperreingriffen. Die Schwellkraft des Bremseingriffs muss hingegen in jedem Sperreingriff unmittelbar am Beginn der Vorschubbewegung von einem Sperreingriff in den nächst folgenden aufgebracht werden. Allerdings soll die Schwellkraft nicht so groß sein, dass sie vom Verwender bei der Ausschüttung als störend empfunden wird.

Eine unerwünschte Vorschubbewegung der Kolbenstange als Reaktion auf die Bewegung des Dosiseinstellglieds 9 bei der Dosisauswahl kann grundsätzlich auch allein durch den Sperreingriff der Blockiereinrichtung 8 bewirkt werden. Allerdings wird solch eine Bewegung wegen des Bremseingriffs sicherer verhindert als durch den Sperreingriff allein.

Die Verbindung zwischen dem Reservoirmodul 10 und dem Dosier- und Betätigungsmodul 30 ist formschlüssig. Zum einen besteht zwischen dem Mechanikhalter 3 und dem Gehäuseabschnitt 11 ein Verriegelungseingriff, der eine Relativbewegung in axialer Richtung verhindert. Über den Verriegelungseingriff hinaus sind der vordere Gehäuseabschnitt 1, 3 und der hintere Gehäuseabschnitt 11 unmittelbar aneinander axial linear geführt, um eine Relativdrehung bei dem Verbinden und im verbundenem Zustand zu verhindern. In Figur 5 sind die Axialführungen 3d des Mechanikhalters 3, die mit einem oder mehreren entsprechenden Eingriffselementen des hinteren Gehäuseabschnitts 11 die Linearführung bilden, gut zu erkennen. Die Axialführungen 3d werden von Führungsflächen an Führungsrippen gebildet; sie könnten auch von Führungsflächen in axial sich erstreckenden Vertiefungen gebildet werden. Auf diese Weise werden axiale Führungskanäle erhalten. Die Führungsrippen sind axial verjüngt, so dass in die Führungskanäle führende Einführtrichter für das eine oder die mehreren Eingriffselemente des hinteren Gehäuseabschnitts 11 gebildet werden. Zur noch besseren Zentrierung der Gehäuseabschnitte 1, 3 und 11 zu Beginn des Verbindens sind die Führungsrippen auch noch in radialer Richtung verjüngt. Das eine oder die mehreren Eingriffselemente des hinteren Gehäuseabschnitts 11 ist oder sind vorzugsweise wie die Axialabschnitte 3d an der Mantelgegenfläche, d.h. der Innenmantelfläche, des hinteren Gehäuseabschnitts 11 gebildet.

Der Verriegelungseingriff besteht zwischen einem weiblichen, ersten Verriegelungselement 3a des Mechanikhalters 3 (Figur 5) und einem Verriegelungsring 20, der mit dem hinteren Gehäuseabschnitt 11 radial bewegbar, aber axial nicht bewegbar verbunden ist. Der Verriegelungsring 20 bildet ein unmittelbar in das erste Verriegelungselement 3a radial eingreifendes männliches, zweites Verriegelungselement 21. Zwischen dem ersten Verriegelungselement 3a und dem zweiten Verriegelungselement 21 besteht eine Schloss/Riegel-Verbindung, die axiale Relativbewegungen zwischen dem Reservoirmodul 10 und dem Dosier- und Betätigungsmodul 30 verhindert.

Die Figuren 3 und 4 zeigen das Verriegelungselement 21 im Verriegelungseingriff mit dem Verriegelungselement 3a. Das Verriegelungselement 3a wird von einem Ringsteg und einer Nut gebildet, die an dem Außenmantel des Mechanikhalters 3 umlaufen. Der Ringsteg bildet eine hintere Seitenwand der Nut. Das zweite Verriegelungselement 21 wird von einem Nocken gebildet, der von dem Innenmantel des Verriegelungsrings 20 radial einwärts vorragt und im Verriegelungseingriff von einer Rückstelleinrichtung 24 radial einwärts über eine Innenmantelfläche des hinteren Gehäuseabschnitts 11 vorstehend in das aufnehmende Verriegelungselement 3a hineingedrückt wird. Der Verriegelungsring 20 ist in seiner Gesamtheit in Radialrichtung mittels der Rückstelleinrichtung 24 an einer von dem Gehäuseabschnitt 11 gebildeten Innenmantelfläche abgestützt, so dass die Rückstelleinrichtung 24 etwa in radialer Verlängerung des Verriegelungselements 21 gegen den Außenmantel des Verriegelungsrings 20 drückt. Der Verriegelungsring 20 umgibt den Mechanikhalter 3 und ist in seiner Gesamtheit gegen die rückstellende Kraft der Rückstelleinrichtung 24 radial hin und her bewegbar, so dass das zweite Verriegelungselement 21 in und aus dem Verriegelungseingriff mit dem ersten Verriegelungselement 3a bringbar ist. Der hintere Gehäuseabschnitt 11 bildet eine enge Gleitführung für die Radialbewegung des Verriegelungsrings 20. An seiner dem Verriegelungselement 21 radial gegenüberliegenden Seite bildet der Verriegelungsring 20 einen Entriegelungsknopf 22 für den Verwender. Zur radialen Führung der als Druckfeder ausgebildeten Rückstelleinrichtung 24 ragt von der dem Verriegelungselement 21 abgewandten Außenmantelfläche des Verriegelungsrings 20 ein Führungsnocken radial ab.

In Umfangsrichtung zu beiden Seiten dieses Führungsnockens und axial hinter dem Führungsnocken ragen von der Außenmantelfläche des Verriegelungsrings 20 ferner zwei Sperrnocken 23 ab, die in Radialrichtung nach außen gegen eine Verriegelungssperre 25 drücken. Aufgrund der Anlage der Sperrnocken 23 gegen die Verriegelungssperre 25 wird eine Radialbewegung des Verriegelungselements 21 verhindert, die zu einem Lösen des Verriegelungseingriffs führen könnte. Der zwischen den Verriegelungselementen 3a und 21 bestehende Verriegelungseingriff wird somit durch die Verriegelungssperre 25 gesichert. Diese Sicherung besteht in jeder Verschiebeposition des Dosier- und Betätigungselements 12 mit Ausnahme einer Freigabestellung, die das Dosier-und Betätigungselement 12 am Ende seiner Ausschüttbewegung einnimmt. Die Freigabestellung fällt daher mit der vordersten Verschiebeposition zusammen, die das Dosier- und Betätigungselement 12 dann einnimmt, wenn es im Zuge seiner Ausschüttbewegung an das Dosiseinstellglied 9 und das Dosiseinstellglied 9 seinerseits gegen den Ausschüttanschlag 3c des Mechanikhalters 3 anstößt. Solange das Dosier- und Betätigungsmodul 30 mit dem Reservoirmodul noch nicht verbunden ist, wird ein mechanischer Anschlag für das Dosier- und Betätigungselement 12 von einem Anschlagelement 31 der Dosier- und Betätigungsvorrichtung gebildet. Im Ausführungsbeispiel bildet ein Resethalterring, der einem Reset der Anzeige 17 dient, das Anschlagelement 31. Der Anschlag des Dosier- und Betätigungselement 12 gegen dieses Anschlagelement 31 definiert die Freigabestellung des Dosier- und Betätigungselements 12 in diesem Falle, wobei die durch das Anschlagelement 31 definierte Freigabestellung derjenigen entspricht, die durch das Anstoßen des Dosiseinstellglieds 9 an dem Ausschüttanschlag 3c definiert wird.

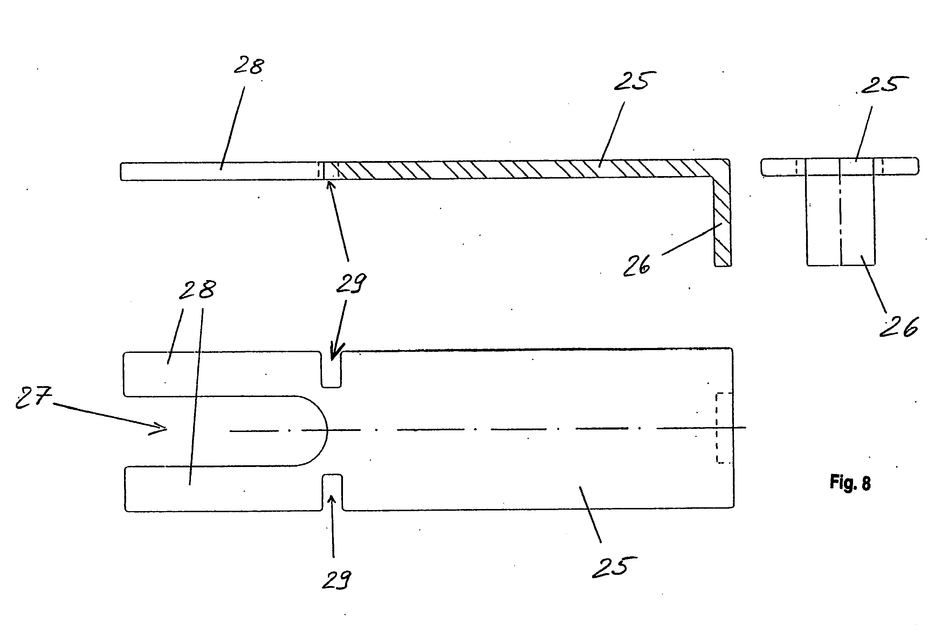

Figur 8 zeigt die Verriegelungssperre 25. Sie wird im Ausführungsbeispiel einstückig von einem Sperrschieber gebildet. Die Verriegelungssperre 25 weist einen plattenförmigen Hauptkörper auf, der sich im montierten Zustand, wie beispielsweise in Figur 4 dargestellt, axial erstreckt. An einem Ende ragt von dem Hauptkörper ein Steg 26 rechtwinklig ab. Im montierten Zustand erstreckt sich der Steg 26 radial bis gegen das Dosier- und Betätigungselement 12. Der Steg 26 dient der Befestigung der Verriegelungssperre 25 an dem Dosier- und Betätigungselement 12, das hierfür zwei axial beabstandet an einer Außenmantelfläche gebildete Ringstege aufweist, die Mitnehmer 15a und 15b bilden. Der vordere Mitnehmer 15b bildet gleichzeitig die Stützschulter für die Rückstelleinrichtung 16. In den zwischen den Mitnehmern 15a und 15b gebildeten Ringraum ragt die Verriegelungssperre 25 mit ihrem Steg 26 ein und wird von den beiden Mitnehmern 15a und 15b axial beidseits eng eingefasst.

An einem von dem Steg 26 abgewandten vorderen Ende ist der Hauptkörper der Verriegelungssperre 25 mit einer Axialausnehmung 27 versehen, die zu dem vorderen Ende der Verriegelungssperre 25 hin offen ist. Auf diese Weise werden beidseits der Ausnehmung 27 axial sich erstreckende Sperrzungen 28 gebildet. Die beiden Sperrnocken 23 des Verriegelungsrings 20 sind so angeordnet, dass je einer dieser Sperrnocken 23 gegen eine der Sperrzungen 28 drückt, solange das Dosier- und Betätigungselement 12 nicht die Freigabestellung einnimmt. Durch die axiale Ausnehmung 27 hindurch erstreckt sich bei der Axialbewegung der Verriegelungssperre 25 die Rückstelleinrichtung 24 für das Verriegelungselement 21.

In dem Hauptkörper der Verriegelungssperre 25 sind ferner Einrückausnehmungen 29 gebildet, die die Freigabestellung des Dosier- und Betätigungselements 12 definieren. Pro Sperrnocken 23 ist je eine Einrückausnehmung 29 vorgesehen. Die Position der Einrückausnehmungen 29 ist so gewählt, dass sie mit den Sperrnocken 23 erst dann in Überdeckung gelangen und dadurch ein Einfahren der Sperrnocken 23 gestatten, wenn das Dosier- und Betätigungselement 12 bis in seine Freigabestellung vorbewegt worden ist.

Es ist klar, dass bei der im Ausführungsbeispiel speziell gewählten Anordnung auch ein einziger Sperrnocken 23 vorgesehen sein könnte und die Verriegelungssperre 25 dementsprechend auch nur eine einzige Einrückausnehmung 29 und gegebenenfalls auch nur eine Sperrzunge 28 aufweisen könnte. Grundsätzlich könnte die Verriegelungssperre ferner einstückig mit dem Dosier- und Betätigungselement 12 gefertigt sein. Die Ausbildung als separates Teil bietet jedoch Vorteile hinsichtlich der Fertigung, der Montage und dem Zusammenwirken des Dosier- und Betätigungselements 12 mit der Kolbenstange 4. In Bezug auf die Einbaulage der Verriegelungssperre 25 ist noch darauf hinzuweisen, dass die Verriegelungssperre 25 an ihrer von dem Verriegelungselement 21 abgewandten Außenseite an einer Innenmantelfläche des Gehäuses 11 abgestützt ist. Auf diese Weise wird die Stabilität der Sicherung für den Verriegelungseingriff erhöht. Vorzugsweise bildet das Gehäuse 11 eine Axialführung für die Verriegelungssperre 25.

Im Folgenden wird die Funktionsweise des Injektionsgeräts beschrieben, wobei angenommen sei, dass ein neues Reservoirmodul 10 und ein bereits wenigstens einmal verwendetes Dosier- und Betätigungsmodul 30 zusammengebaut und anschließend eine erste Produktausschüttung vorgenommen werden.

Das Dosier- und Betätigungsmodul 30 und das neue Reservoirmodul 10 werden axial zueinander ausgerichtet, so dass ihre beiden Längsachsen miteinander fluchten. Anschließend wird das Reservoirmodul 10 mit seinem rückwärtigen Ende in das nach vorne offene Gehäuse 11 des Dosier- und Betätigungsmoduls 30 eingeführt.

Hierbei zentrieren sich der Gehäuseabschnitt 1, 3 und der Gehäuseabschnitt 11 an den verjüngten Enden der Führungsrippen 3d des Mechanikhalters 3. Während des Aufschiebens werden die beiden Gehäuseabschnitte in einer durch die Linearführung vorgegebenen Drehwinkelposition axial aneinander lineargeführt, bis die Gehäuseabschnitte 1, 3 und 11 eine Verbindungsendposition einnehmen, in der der Verriegelungseingriff der Verriegelungselemente 3a und 21 hergestellt werden kann oder von selbst sich einstellt.

Das Dosier- und Betätigungselement 12 ist in vorgegebenen Drehwinkelpositionen relativ zu dem hinteren Gehäuseabschnitt 11 gerastet. Die Linearführung der Gehäuseabschnitte 1, 3 und 11 und die Drehwinkelrastposition des Dosier- und Betätigungselements 12 sind so aufeinander abgestimmt, dass der verdrehgesicherte Eingriff des Dosier- und Betätigungselements 12 mit der Kolbenstange 4 in jeder Rastposition des Dosier- und Betätigungselements 12 und jeder Drehwinkelposition, in der die Gehäuseabschnitte 1, 3 und 11 aneinander lineargeführt sind, hergestellt wird.

Falls das Dosier- und Betätigungselement 12 sich in einer Axialposition relativ zu dem Gehäuseabschnitt 11 befindet, die hinter der Freigabestellung liegt, wird das Verriegelungselement 21 von der Verriegelungssperre 25 in seiner radial innersten Stellung gehalten. In dieser Stellung des Verriegelungselements 21 können das Dosier- und Betätigungsmodul 30 und das Reservoirmodul 10 nicht bis in die Verbindungsendstellung aufeinandergeschoben und deshalb auch nicht miteinander verbunden werden, da der am Außenmantel des Mechanikhalters 3 gebildete Ringsteg, der das erste Verriegelungselement 3a mitbildet, vorher gegen das zweite Verriegelungselement 21 auf Anschlag zu liegen kommt.

Der Ringsteg kann zu einem in tangentialer Richtung kurzen Radialvorsprung reduziert werden, wenn dafür gesorgt ist, dass die Gehäuseabschnitte 1, 3 und 11 nur in der Drehwinkellage zusammengebaut werden können, in der solch ein Vorsprung und das zweite Verriegelungselement 21 in einer axialen Flucht zu liegen kommen. Der Ringsteg oder Radialvorsprung könnte das erste Verriegelungselement 3a auch alleine bilden, da es die wesentliche Funktion des ersten Verriegelungselements 3a ist, die Herstellung der Verbindung zwischen dem Reservoirmodul 10 und dem Dosier- und Betätigungsmodul 30 nur zuzulassen, wenn das Dosier- und Betätigungselement 12 seine Freigabestellung einnimmt. Ist diese Bedingung erfüllt, so würde das Dosier- und Betätigungselement 12 bei der Herstellung der Verbindung zwischen dem Reservoirmodul 10 und dem Dosier- und Betätigungsmodul 30 sicherstellen, dass das Dosiseinstellglied 9 sich in seiner Dosiernullstellung befindet, in der es an dem Ausschüttanschlag 3c des Mechanikhalters 3 anstößt.

Um den die vorstehend erläuterte Bedingung erfüllenden Zustand herzustellen, drückt der Verwender das Dosier- und Betätigungselement 12 axial relativ zu dem hinteren Gehäuseabschnitt 11 bis in die Freigabestellung vor. In dieser Relativstellung zwischen Gehäuseabschnitt 11 und Dosier- und Betätigungselement 12 können die Sperrnocken 23 in die Einrückaufnahmen 29 der Verriegelungssperre 25 bewegt werden. Der Verwender drückt daher nicht nur das Dosier- und Betätigungselement 12 bis wenigstens in die Freigabestellung vor, sondern gleichzeitig auch mittels des Entriegelungsknopfs 22 das erste Verriegelungselement 20 aus dem Verriegelungseingriff. Das Reservoirmodul 10 kann nun axial über den Ringsteg des ersten Verriegelungselements 3a bewegt und weiter in den hinteren Gehäuseabschnitt 11 eingeführt werden. Der Verwender kann den Entriegelungsknopf 22 loslassen. Sobald das erste Verriegelungselement 21 mit dem zweiten Verriegelungselement 3a in Überdeckung gelangt, schnappt es aufgrund der Kraft der Rückstelleinrichtung 24 in das aufnehmende Verriegelungselement 3a ein, so dass der Verriegelungseingriff hergestellt ist. Das Reservoirmodul 10 und das Dosier- und Betätigungsmodul 30 sind nun in Bezug auf die Stellung des Dosiseinstellglieds 9 und der Kolbenstange 4 in definierter Weise miteinander verbunden. Falls das Dosiseinstellglied 9 vor Herstellung des Verriegelungseingriffs noch einen lichten Abstand von dem Ausschüttanschlag 3c aufgewiesen hat, ist dieser Abstand aufgrund der zum Herstellen der Verbindung erforderlichen Einwirkung des Dosier- und Betätigungselements 12 beseitigt. Eine damit einhergehende Produktausschüttung kann hingenommen werden und kann zum Zwecke des Primens der Injektionsnadel sogar erwünscht sein. Die Zähl- und Anzeigeeinrichtung 17 wird vorzugsweise dabei genullt.

In dem derart herbeigeführten, definierten Ausgangszustand kann der Verwender die Dosierung vornehmen. Die Dosierung erfolgt durch Drehung des Dosier- und Betätigungselements 12 um die Längsachse L und relativ zu dem Gehäuseabschnitt 11. Da der Dosiermitnehmer 13 mit dem Dosier- und Betätigungselement 12 verdrehgesichert verbunden ist und seinerseits verdrehgesichert in die Kolbenstange 4 eingreift, nimmt das Dosier- und Betätigungselement 12 bei der rotatorischen Dosierbewegung die Kolbenstange 4 mit. Aufgrund des Gewindeeingriffs zwischen der Kolbenstange 4 und dem Dosiseinstellglied 9 und der Linearführung des Dosiseinstellglieds 9 durch den Mechanikhalter 3 führt das Dosiseinstellglied 9 eine von der Gewindesteigung des gegenseitigen Gewindeeingriffs vorgegebene, axiale translatorische Dosierbewegung in Richtung auf das Dosier- und Betätigungselement 12 aus. Das Dosier- und Betätigungselement 12 bildet einen hinteren Translationsanschlag 12c, der die translatorische Dosierbewegung des Dosiseinstellglieds 9 begrenzt und dadurch den maximal einstellbaren Ausschütthub definiert.

Die Zähl- und Anzeigeeinrichtung 17 zählt die der Drehwinkelstellung des Dosier-und Betätigungselements 12 entsprechenden Dosiseinheiten und zeigt sie optisch an.

Nach Auswahl der gewünschten Produktdosis ist der Dosiervorgang beendet. Die Ausschüttung der gewählten Produktdosis wird mittels der in Vorschubrichtung des Kolbens weisenden Ausschüttbewegung des Dosier- und Betätigungselements 12 bewirkt. Im Zuge seiner Ausschüttbewegung stößt das Dosier- und Betätigungselement 12 gegen das Dosiseinstellglied 9 und nimmt es mit. Indem das Dosiseinstellglied 9 im Zuge der Ausschüttbewegung gegen den Ausschüttanschlag 3c des Mechanikhalters 3 stößt, werden die Ausschüttbewegungen des Dosier- und Betätigungselements 12 und die Produktausschüttung beendet. Nach dem Loslassen des Dosier- und Betätigungselements 12 vorzugsweise wird dieses von dem Rückstelleinrichtung 16 entgegen der Vorschubrichtung wieder in eine neue Ausgangsstellung für eine erneute Dosierung und Produktausschüttung bewegt. Die Zähl- und Anzeigeeinrichtung 17 ist mit dem Dosier- und Betätigungselement 12 vorzugsweise so gekoppelt, dass sie mittlerweile wieder auf Null zurückgestellt worden ist. Gegebenenfalls verfügt sie über Mittel zum Zählen und Anzeigen der bereits insgesamt ausgeschütteten Produktmenge und somit der in der Ampulle 2 verbliebenen Produktrestmenge.

Um das Reservoirmodul 10 von dem Dosier- und Betätigungsmodul 30 zu lösen, wird das Dosier- und Betätigungselement 12 bis in die Freigabestellung, d.h. bis auf Anschlag gegen das Dosiseinstellglied 9, vorbewegt. In dieser Stellung kann der Verwender durch Druck auf den Entriegelungsknopf 22 den Verriegelungseingriff wieder lösen und das Reservoirmodul 10 von dem Dosier-und Betätigungsmodul 30 trennen.

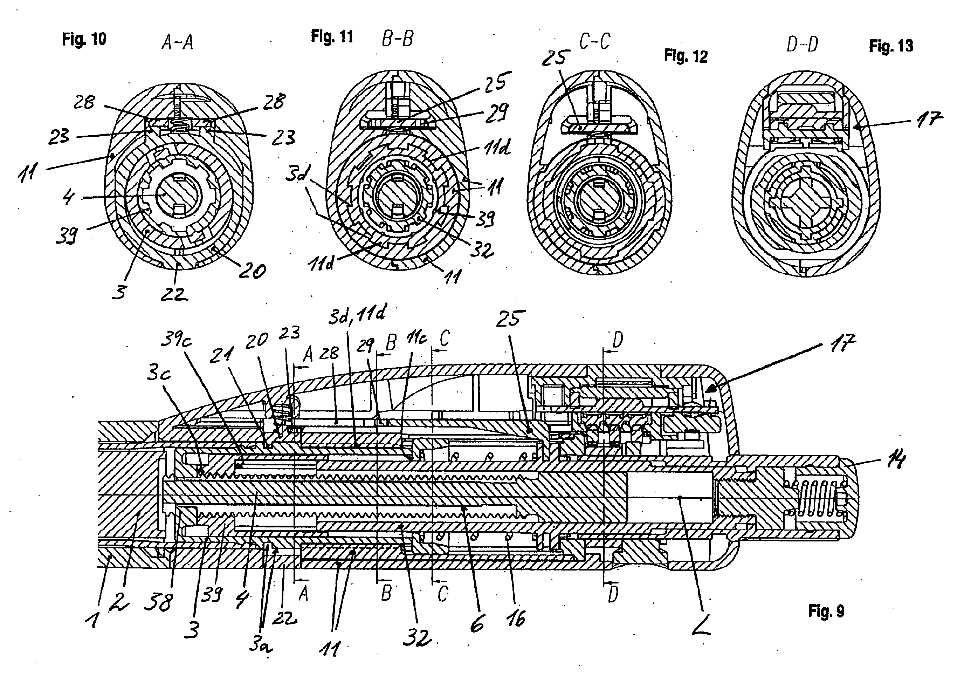

Die Figuren 9 bis 13 zeigen in einem Längsschnitt und vier Querschnitten ein zweites Ausführungsbeispiel eines Injektionsgeräts. Das Injektionsgerät des zweiten Ausführungsbeispiels gleicht demjenigen des ersten Ausführungsbeispiels in Bezug auf die Verriegelung und die Verriegelungssperre 25 derart, dass auf die diesbezügliche Beschreibung des ersten Ausführungsbeispiels verwiesen wird. Insbesondere ist die Verriegelungssperre 25 des zweiten Ausführungsbeispiels in Bezug auf alle funktionalen Details mit derjenigen des ersten Ausführungsbeispiels identisch. Das gleiche gilt für die Verriegelungselemente 3a und 21.

Der Verriegelungsring 20 und die Lage der Sperrnocken 23 relativ zu dem Verriegelungselement 21 und relativ zu der Verriegelungssperre 25 in dem Ausgangszustand des Geräts sind besonders gut in den Querschnitten der Figuren 10, 11 und 12 zu erkennen, auf die diesbezüglich auch stellvertretend für das erste Ausführungsbeispiel verwiesen wird.

Das Injektionsgerät des zweiten Ausführungsbeispiels unterscheidet sich von dem ersten Ausführungsbeispiel durch den Eingriff und den Bewegungsablauf der an der Dosierung beteiligten Komponenten. Ferner erfüllt der Mechanikhalter über die Funktionen des Mechanikhalters des ersten Ausführungsbeispiels hinaus insbesondere die Funktion der Positionierung des Dosiseinstellglieds in diskreten Drehwinkelpositionen, die relativ zu dem Mechanikhalter zum Zwecke der Dosierung veränderbar sind. Die Blockiereinrichtung des zweiten Ausführungsbeispiels ist hingegen einfacher ausgeführt als die des ersten Ausführungsbeispiels. Im Folgenden werden in erster Linie nur die Unterschiede im Vergleich zum ersten Ausführungsbeispiel beschrieben, wobei für Komponenten, die ihrer grundsätzlichen Funktion nach den gleichnamigen Komponenten des ersten Ausführungsbeispiels gleichen, aber in Details unterscheiden, 30iger Zahlen mit gleicher Endziffer oder exakt die gleichen Bezugszeichen, wie bei dem ersten Ausführungsbeispiel, verwendet werden. Soweit zum zweiten Ausführungsbeispiel keine Ausführungen gemacht werden, sollen die entsprechenden Ausführungen zum ersten Ausführungsbeispiel gelten.

In dem zweiten Ausführungsbeispiel ist das relativ zu dem hinteren Gehäuseabschnitt 11 axial linear bewegbare und um die Längsachse L verdrehbare Dosier- und Betätigungselement 32 mit dem Dosiseinstellglied 39 verdrehgesichert verbunden. Das Dosier- und Betätigungselement 32 und das Dosiseinstellglied 39 sind relativ zueinander und relativ zu den Gehäuseabschnitten 1, 3 und 11 in und gegen die Vorschubrichtung bewegbar. Die Kolbenstange 4 wird von dem Mechanikhalter 3 verdrehgesichert gehalten. Die mit dem ersten Ausführungsbeispiel funktionsgleiche Rückzugsperreinrichtung 6 verhindert im Zusammenwirken mit Sperrelementen der einstückig an dem Mechanikhalter 3 geformten Blockiereinrichtung 38 eine Bewegung der Kolbenstange 4 entgegen der Vorschubrichtung, lässt eine Bewegung in die Vorschubrichtung jedoch zu. Die Sperrelemente bilden gleichzeitig die Rückzugsperre und die Verdrehsicherung für die Kolbenstange 4. Des Weiteren bildet wie bereits im ersten Ausführungsbeispiel das Dosier- und Betätigungselement 32 eine Gleitführung für die Kolbenstange 4.

Bei der Dosierung führt das Dosier- und Betätigungselement 32 die gleiche rotatorische Dosierbewegung wie das Dosier- und Betätigungselement 12 des ersten Ausführungsbeispiels aus. Allerdings wird aufgrund des verdrehgesicherten Eingriffs das Dosiseinstellglied 39 bei der rotatorischen Dosierbewegung mitgenommen. Der Gewindeeingriff zwischen der Kolbenstange 4 und dem Dosiseinstellglied 39 ist wieder mit demjenigen des ersten Ausführungsbeispiels vergleichbar, so dass ein von dem Dosiseinstellglied 39 gebildeter Anschlag 39c aufgrund der rotatorischen Dosierbewegung und des Gewindeeingriffs mit der Kolbenstange 4 im Zuge der Dosierung entgegen der Vorschubrichtung auf ein vorderes Ende des Dosier- und Betätigungselements 32 zu bewegt wird. Im Gegensatz zum ersten Ausführungsbeispiel vollführt das Dosiseinstellglied 39 somit bei der Dosierung eine rotatorische Dosierbewegung und eine translatorische Dosierbewegung relativ zu dem vorderen Gehäuseabschnitt, während die Kolbenstange 4 stillsteht. Durch die nach Abschluss der Dosierung erfolgende Ausschüttbewegung des Dosier- und Betätigungselements 32 wird die Kolbenstange 4 um die Weglänge vorgeschoben, die dem durch die Dosierung eingestellten lichten Abstand zwischen einer Anschlagfläche des Dosiseinstellglieds 39 und dem Ausschüttanschlag 3c des Mechanikhalters 3 entspricht.

Die translatorische Dosierbewegung des Dosiseinstellglieds 39 wird gegen die Vorschubrichtung von einem hinteren Translationsanschlag 11 c begrenzt, den der hintere Gehäuseabschnitt 11 selbst unmittelbar bildet. Auch bei dem zweiten Ausführungsbeispiel bildet die Längsachse L die Rotations- und Translationsachse der Komponenten, die an der Dosierung und Produktausschüttung beteiligt sind.

Der vordere Gehäuseabschnitt 1, 3 bildet wie bei dem ersten Ausführungsbeispiel eine Gleitführung für das Dosiseinstellglied 39. Zur Ausbildung der Gleitführung stehen eine innere Mantelfläche des Mechanikhalters 3 und eine äußere Mantelfläche des Dosiseinstellglieds 39 miteinander in Gleitkontakt. Das Dosier-und Betätigungselement 32 steht mit einer Innenmantelfläche des Dosiseinstellglieds 39 in einem Eingriff, um die verdrehgesicherte Verbindung zwischen dem Dosiseinstellglied 39 und dem Dosier- und Betätigungselement 32 zu bilden.

In dem zweiten Ausführungsbeispiel weist die Kolbenstange 4 über die Rückzugssperreinrichtung 6 hinaus keine eigene Bremseinrichtung auf. Die Vorderseiten der Sägezähnen der Rückzugssperreinrichtung 6 bilden vielmehr alleine auch die Bremseinrichtung. Die Kolbenstange 4 des zweiten Ausführungsbeispiels kann jedoch durch die Kolbenstange des ersten Ausführungsbeispiels ersetzt werden. Entsprechend wären in diesem Falle durch den Mechanikhalter 3 des zweiten Ausführungsbeispiels auch wenigstens ein Bremselement, vorzugsweise beide Bremselemente des ersten Ausführungsbeispiels auszubilden.

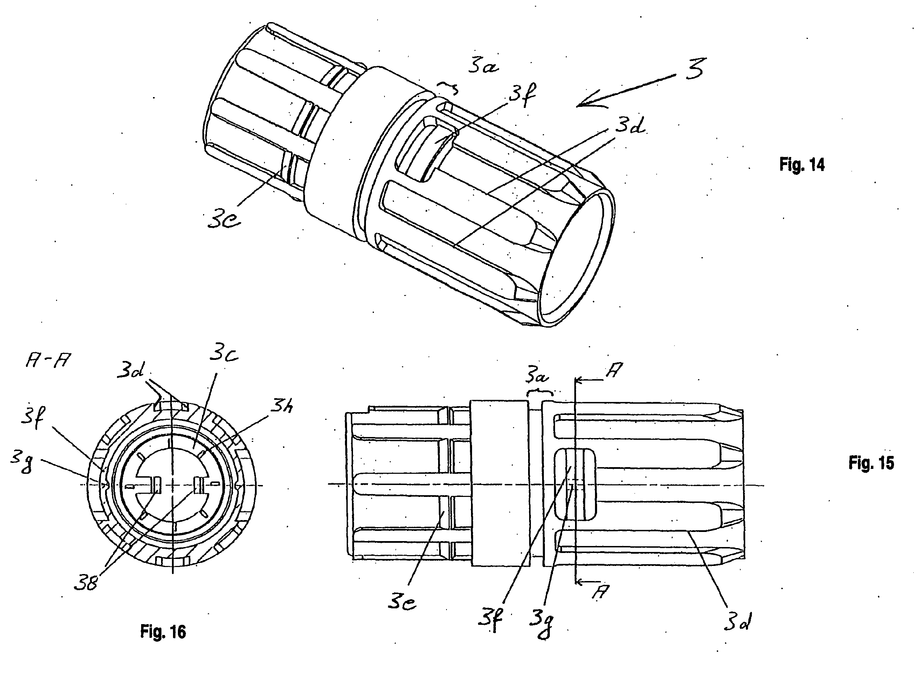

Die Figuren 14 bis 16 zeigen den Mechanikhalter 3 des zweiten Ausführungsbeispiels in einer perspektivischen Darstellung, einer Seitenansicht und in dem in der Seitenansicht eingetragenen Querschnitt A-A. Der Mechanikhalter 3 ist wie beim ersten Ausführungsbeispiel als einteiliges Hülsenteil ausgeführt, vorzugsweise als Kunststoffspritzgussteil. Er weist an dem Außenmantel eines vorderen Hülsenabschnitts einen Wulst 3e auf. Der vordere Hülsenabschnitt wird in das Reservoirteil 1 eingesteckt und mittels des Wulstes 3e zumindest für den Verwender unlösbar mit dem Reservoirteil 1 verrastet.

Das Verriegelungselement 3a ist an einem mittleren Hülsenabschnitt des Mechanikhalters 3 wie bei dem ersten Ausführungsbeispiel ausgebildet.

Ein hinterer Hülsenabschnitt, der sich an das Verriegelungselement 3a anschließt, bildet an seinem Außenumfang eine Mehrzahl von Axialführungen 3d. Die Axialführungen 3d werden von Führungsrippen gebildet, die an dem Außenumfang des hinteren Hülsenabschnitts radial aufragen. Genauer gesagt wird die Axialführung von den axial sich erstreckenden, geraden Seitenwänden dieser Führungsrippen gebildet, so dass, wie bei dem ersten Ausführungsbeispiel, axiale Führungskanäle erhalten werden. Die Führungsrippen ragen von dem mittleren Hülsenabschnitt aus wie Finger bis an das hintere Ende des Mechanikhalters 3, wo sie axial verjüngt auslaufen. Die Axialführung 3d dient der Linearführung des hinteren Gehäuseabschnitts 11 bei dem Verbinden des Reservoirmoduls 10 mit dem Dosier- und Betätigungsmodul 30. Wie in Figur 9 und am besten in Figur 11 zu erkennen ist ragen von einer Innenmantelfläche des hinteren Gehäuseabschnitts 11 der Anzahl nach entsprechend und der Form nach angepasst Eingriffselemente 11d radial einwärts ab. In jede der Axialführungen 3d ragt ein Eingriffselement 11d ein und wird von der Axialführung 3d linear geführt, wenn der vordere Gehäuseabschnitt 1, 3 und der hintere Gehäuseabschnitt 11 zum Verbinden ineinander geschoben werden. Auf diese Weise wird sichergestellt, dass zwischen dem vorderen Gehäuseabschnitt 1, 3 und dem hinteren Gehäuseabschnitt 11 keine Relativdrehung stattfinden kann, wenn im Zuge des Verbindens der verdrehgesicherte Eingriff zwischen dem Dosier- und Betätigungselement 32 und dem Dosiseinstellglied 39 hergestellt wird.

Indem die Führungsrippen an ihren hinteren Enden axial verjüngt auslaufen und die Führungskanäle so zu Einführtrichtern verbreitert sind, wird eine Zentrierung zwischen dem vorderen Gehäuseabschnitt 1, 3 und dem hinteren Gehäuseabschnitt 11 zum Zwecke des Verbindens erleichtert. Die Führungsrippen laufen an ihren Enden auch radial zur Mantelfläche des Mechanikhalters 3 verjüngt aus, wodurch die Zentrierung der Gehäuseabschnitte 1, 3 und 11 in eine durch die Axialführung 3d vorgegebene Drehwinkelposition relativ zueinander noch mehr erleichtert wird.

Ebenso wie bei dem Ineinanderschieben der vordere Gehäuseabschnitt 1, 3 und der hintere Gehäuseabschnitt 11 an einer Relativdrehung zueinander gehindert werden, wird auch das Dosiseinstellglied 39 in Bezug auf seine Drehwinkelposition relativ zu dem vorderen Gehäuseabschnitt 1, 3 fixiert, wobei diese Fixierung lösbar ist, um die für die Dosierung erforderliche Drehbewegung des Dosiseinstellglieds 39 zuzulassen. Um daher zum einen die Dosierbewegung des Dosiseinstellglieds 39 zu ermöglichen, aber zum anderen eine unerwünschte Dosierbewegung durch das Herstellen der Verbindung zwischen dem vorderen Gehäuseabschnitt 1, 3 und dem hinteren Gehäuseabschnitt 11 zu verhindern, wird das Dosiseinstellglied 39 von dem Mechanikhalter 3 mittels einer lösbaren Rastverbindung in diskreten Drehwinkelpositionen fixiert.

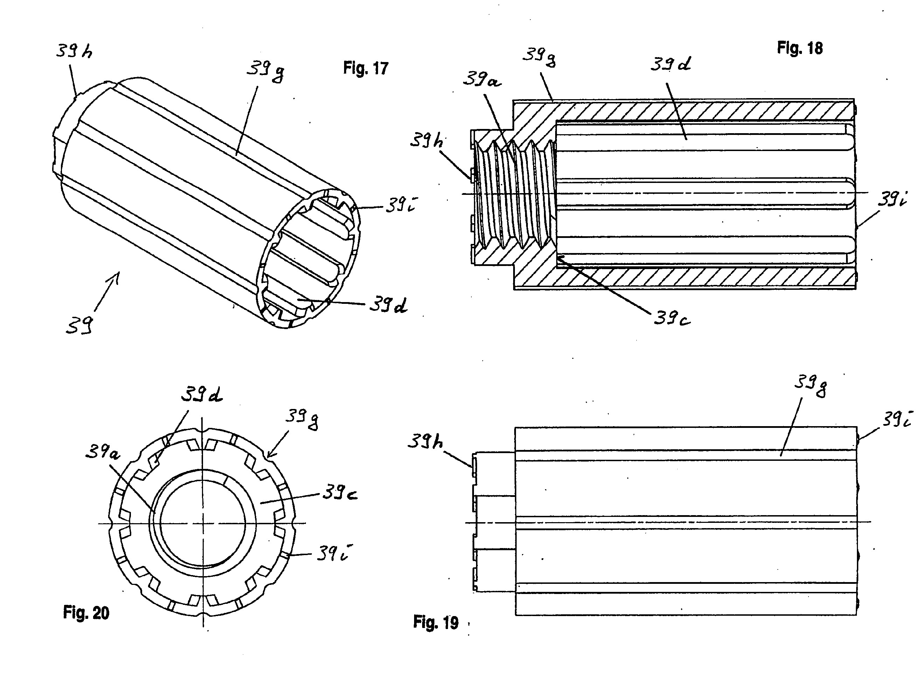

Die Figuren 17 bis 20 zeigen das Dosiseinstellglied 39 in Einzeldarstellungen. Für die Ausbildung der Rastverbindung sind an der Außenmantelfläche des Dosiseinstellglieds 39 über den Umfang in regelmäßiger Teilung verteilt mehrere Rastaufnahmen 39g ausgebildet. Jede der Rastaufnahmen 39g wird von einer geraden, axial sich erstreckenden Rinne mit einer im Querschnitt gerundet verlaufenden Kontur gebildet.

Der Mechanikhalter 3 ist mit zwei Rastnasen 3g (Figur 15 und 16) versehen. Die beiden Rastnasen 3g ragen in dem hinteren Hülsenabschnitt des Mechanikhalters 3 von einer Innenmantelfläche des Mechanikhalters 3 radial einwärts ab. Sie sind einander diametral gegenüberliegend angeordnet. Der jeweilige Mantelbereich des Mechanikhalters 3, an dem eine der Rastnasen 3g angeformt ist, bildet ein in radialer Richtung elastisch nachgiebiges Federelement 3f. Aufgrund der elastischen Nachgiebigkeit und der gerundeten Form der Rastnasen 3g in Verbindung mit dem gerundeten Profil der Rastaufnahmen 39g ist der Rasteingriff der Rastnasen 3g in die gegenüberliegenden Rastaufnahmen 39g lösbar. Die Lösbarkeit ist für die Dosisauswahl erforderlich. Andererseits ist der Rasteingriff jedoch so gestaltet, dass die Drehwinkelfixierung des Dosiseinstellglieds 39 so stabil ist, dass bei dem Verbinden des vorderen Gehäuseabschnitts 1, 3 mit dem hinteren Gehäuseabschnitt 11 keine unerwünschte Dosierbewegung des Dosiseinstellglieds 39 stattfinden kann, wenn die Drehkopplung des Dosier- und Betätigungselements 32 mit dem Dosier- und Betätigungselement 32 hergestellt wird. Die Rastverbindung zwischen dem Mechanikhalter 3 und dem Dosiseinstellglied 39 ergibt als vorteilhaften Nebeneffekt ein taktiles Signal bei der Dosierung. Um die günstige Elastizität des Federelements 3f zu erhalten, ist der hintere Hülsenabschnitt des Mechanikhalters 3 im betreffenden Mantelbereich ausgeschnitten, so dass das Federelement 3f als ein im Umfangsrichtung sich erstreckendes Ringsegment erhalten wird, das axial beidseits freiliegt.

Aus den Figuren 17, 18 und 20 ebenfalls erkennbar sind Axialführungen 39d für den verdrehsicheren Eingriff des Dosiseinstellglieds 39 mit dem Dosier- und Betätigungselement 32. Das Dosier- und Betätigungselement 32 ist mit wenigstens einem Eingriffselement versehen, um die axiale Linearführung, d.h. die Verdrehsicherung, zwischen dem Dosier- und Betätigungselement 32 und dem Dosiseinstellglied 39 zu erhalten. Die Axialführungen 39d sind wieder Führungskanäle, die von mehreren, axial sich gerade erstreckenden Führungsrippen gebildet werden. Jede der Führungsrippen läuft an ihrem hinteren, dem Dosier- und Betätigungselement 32 zugewandten Ende axial und radial verjüngt aus, um die Zentrierung zwischen dem Dosier- und Betätigungselement 32 und dem Dosiseinstellglied 39 bei der Herstellung des verdrehsicheren Eingriffs zu erleichtern. Für die axiale Linearführung von Dosiseinstellglied 39 und Dosier-und Betätigungselement 32 wird somit die gleiche Konstruktion wie für die axiale Linearführung der Gehäuseabschnitte 1, 3 und 11 verwendet.

Der Vollständigkeit wegen sei schließlich auch auf das Dosiergewinde 39a und den Ausschüttanschlag 39c des Dosiseinstellglieds 39 hingewiesen, die am besten in Figur 18 zu erkennen sind.

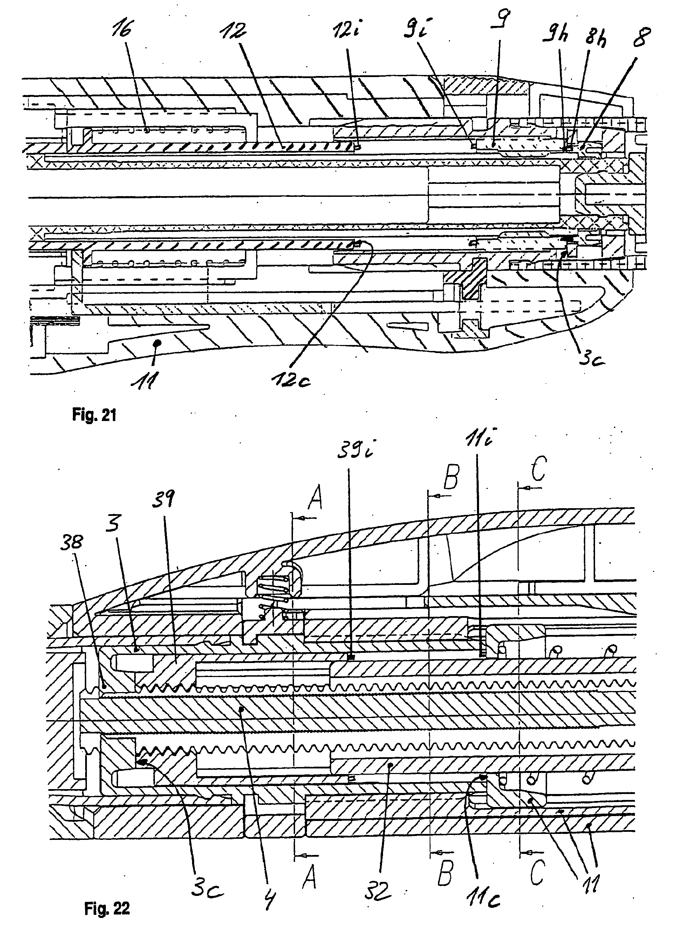

Schließlich sind für das Dosiseinstellglied 39 zwei Verdrehsicherungen vorgesehen, die in den beiden axialen Endpositionen des Dosiseinstellglieds 39 wirksam werden. Diesbezüglich sei ergänzend auf Figur 22 hingewiesen.

Um zu verhindern, dass die Kolbenstange 4 in Reaktion auf eine rotatorische Dosierbewegung des Dosiseinstellglieds 39 zurückbewegt werden könnte, sind an einem vorderen Ende des Dosiseinstellglieds 39 Verdrehanschläge 39h geformt. Die Verdrehanschläge 39h stehen in der vorderen Endposition, die das Dosiseinstellglied 39 unmittelbar nach einer Produktausschüttung bzw. vor einer Dosisauswahl einnimmt, mit Verdrehgegenanschlägen 3h in Eingriff, die an dem Mechanikhalter 3 angeformt sind (Fig. 16). Die Verdrehanschläge 39h ragen von einer vorderen Stirnseite des Dosiseinstellglieds 39 axial ab, und die Verdrehgegenanschläge 3h ragen von einer axial zugewandten Stirnfläche des Mechanikhalters 3, die den Aufschüttanschlag 3c bildet, den Verdrehanschlägen 39h axial entgegen. Der Eingriff zwischen den Verdrehanschlägen 39h und den Verdrehgegenanschlägen 3h ist derart, dass er eine rotatorische Dosierbewegung in eine Drehrichtung zulässt, die eine von dem Ausschüttanschlag 3c weg gerichtete translatorische Dosierbewegung des Dosiseinstellglieds 39 bewirkt, aber in der vorderen axialen Endposition eine rotatorische Dosierbewegung in die Gegendrehrichtung verhindert.

Ferner ist ein weiteres Paar von Verdrehanschlägen und Verdrehgegenanschlägen vorgesehen, die in grundsätzlich gleicher Weise wie die Anschläge 3h und 39h geformt sind und zusammenwirken. Bei diesem zweiten Verdrehanschlagpaar handelt es sich zum einen um Verdrehanschläge 39i, die von einer hinteren Stirnfläche des Dosiseinstellglieds 39 axial abragen, und zum anderen um Verdrehgegenanschläge 11 i, die von der zugewandten Anschlagstirnfläche des hinteren Translationsanschlags 11c axial auf das Dosiseinstellglied 39 zuragen, in der Figur 9 aufgrund ihrer geringen Abmessungen jedoch nicht erkennbar sind. Durch das hintere Verdrehanschlagpaar 11 i/39i wird in der hinteren Endposition verhindert, dass die Kolbenstange 4 in Reaktion auf eine gegen die Anschlagstirnfläche des hinteren Translationsanschlags 11 c gerichtete Dosierbewegung des Dosiseinstellglieds in Vorschubrichtung bewegt werden kann.
Die Höhe, d.h. die axiale Länge, sämtlicher Verdrehanschläge 3h, 39h, 11i und 39i ist auf die Gewindesteigung der im Eingriff befindlichen Dosiergewinde der Kolbenstange 4 und des Dosiseinstellglieds 39 abgestimmt. Die Verdrehanschläge sind axial so kurz, dass diejenige rotatorische Dosierbewegung nicht behindert wird, durch die das Dosiseinstellglied 39 von dem jeweiligen Translationsanschlag 3c oder 11 c wegbewegt wird.

Bei der Montage der Komponenten des Reservoirmoduls 10 wird das Dosiseinstellglied 39 bis in eine vorgegebene Axialposition, wie sie aus Figur 9 ersichtlich ist, auf die Kolbenstange 4 aufgeschraubt. Anschließend wird die Kolbenstange 4 mit dem aufgeschraubten Dosiseinstellglied 39 von hinten in den Mechanikhalter 3 eingeführt, bis dessen Blockiereinrichtung 38 in Sperreingriff mit der Rückzugssperreinrichtung 6 der Kolbenstange 4 gelangt und ferner der verdrehgesicherte Eingriff zwischen den Verdrehanschlägen 39h des Dosiseinstellglieds 39 und den Verdrehgegenanschlägen 3h des Mechanikhalters 3 hergestellt ist. Bereits während der Einführung in den Mechanikhalter 3 wird das Dosiseinstellglied 39 in einer Drehwinkelrastposition durch den Rasteingriff zwischen den Rastnasen 3g und den Rastaufnahmen 39g von dem Mechanikhalter 3 axial linear geführt, bis das Dosiseinstellglied 39 an den Ausschüttanschlag 3c des Mechanikhalters 3 anstößt. In dieser vorderen Endposition des Dosiseinstellglieds 39 relativ zu dem Mechanikhalter 3 ist gleichzeitig auch bereits der verdrehsichere Eingriff zwischen den Verdrehanschlägen 3h und 39h hergestellt.

In diesem Zustand werden der Mechanikhalter 3 und ein bereits mit einem Reservoir ausgestattetes Reservoirteil 1 miteinander verbunden.

In einem nächsten Schritt wird der hintere Gehäuseabschnitt 11 des komplett montierten Dosier- und Betätigungsmoduls 30 auf den Mechanikhalter 3 geschoben, wobei sich der Mechanikhalter 3 und der hintere Gehäuseabschnitt 11 aufgrund der Axialführungen 3d und der Eingriffselemente 11d des hinteren Gehäuseabschnitts 11 zueinander zentrieren können und nach der Zentrierung aufgrund des Führungseingriffs axial aneinander linear geführt werden. Im Zuge des Aufschiebens gelangt das Dosier- und Betätigungselement 32 in den verdrehgesicherten Eingriff mit dem Dosiseinstellglied 39, wobei auch hier durch eine den Axialführungen 3d und Eingriffselementen 11d entsprechende Linearführung zuerst eine gewisse Zentrierung stattfinden kann.

Das Dosier- und Betätigungselement 32 ist in diskreten Drehwinkelrastpositionen mit dem hinteren Gehäuseabschnitt in einem Rasteingriff und wird in dem Rasteingriff, d.h. in der jeweiligen Drehwinkelrastposition, axial linear geführt. Die Drehwinkeldifferenz zwischen zwei aufeinanderfolgenden Drehwinkelrastpositionen entspricht einer Dosiseinheit. Die Linearführung zwischen dem Mechanikhalter 3 und dem hinteren Gehäuseabschnitt 11 einerseits und die diskreten Drehwinkelpositionen des Dosiseinstellglieds 39 relativ zu dem Mechanikhalter 3 (Rastnasen 3g und Rastaufnahmen 39g) und die Drehwinkelrastpositionen des Dosier- und Betätigungselements 32 relativ zu dem hinteren Gehäuseabschnitt 11 andererseits sind aufeinander so abgestimmt, dass ein lineares Übereinanderschieben der beiden Gehäuseabschnitte 1, 3 und 11 stets in solch einer Drehwinkelposition stattfindet, dass auch das Dosiseinstellglied 39 und das Dosier- und Betätigungselement 32 für ihren verdrehgesicherten Eingriff relativ zueinander ausgerichtet sind, so dass während des Verbindens des Reservoirmoduls 10 mit dem Dosier- und Betätigungsmodul 30 keine Relativdrehung zwischen den an der Dosierung beteiligten Komponenten stattfindet.

In Bezug auf die weiteren Details der Montage, insbesondere der Herstellung des Verriegelungseingriffs, und zur Funktionsweise des Injektionsgeräts nach dem zweiten Ausführungsbeispiel wird auf die Beschreibung zu dem ersten Ausführungsbeispiel verwiesen.

Auch bei dem Injekionsgerät nach dem ersten Ausführungsbeispiel können Verdrehsicherungen vorgesehen sein, die in den beiden axialen Endpositionen des Dosiseinstellglieds 9 des ersten Ausführungsbeispiels unerwünschte Reaktionsbewegungen der Kolbenstange 4 verhindern. Figur 21 zeigt die beiden Verdrehsicherungen, die in gleicher Weise wie die Verdrehsicherungen des zweiten Ausführungsbeispiels geformt sind. Allerdings werden die bei dem zweiten Ausführungsbeispiel an den Gehäuseabschnitten 1, 3 und 11 geformten Verdrehgegenanschläge bei dem ersten Ausführungsbeispiel zum einen von der Blockiereinrichtung 8 und zum anderen von dem Dosier- und Betätigungselement 12 gebildet. So sind an der Stirnfläche der Blockiereinrichtung 8, die dem Dosiseinstellglied 9 axial zugewandt ist, mehrere Verdrehanschläge 8h angeformt, die auf das Dosiseinstellglied 9 axial zu ragen. Da die Blockiereinrichtung 8 von dem vorderen Gehäuseabschnitt 1, 3 axial unbeweglich gelagert wird und mit der Kolbenstange 4 verdrehgesichert verbunden ist, wird auch durch das vordere Verdrehanschlagpaar 8h/9h eine Verdrehsicherung für die zwischen der Kolbenstange 4 und dem Dosiseinstellglied 9 stattfindende rotatorische Dosierbewegung erhalten. Das zweite Verdrehanschlagpaar ist zwischen dem Dosiseinstellglied 9 und dem hinteren Translationsanschlag 12c gebildet. Wie bei dem zweiten Ausführungsbeispiel ragen von der Stirnfläche des Translationsanschlags 12c, die dem Dosiseinstellglied 9 axial zugewandt ist, mehrere Verdrehanschläge 12i axial in Richtung auf das Dosiseinstellglied 9 vor. Das Dosiseinstellglied 9 ist wie bei dem zweiten Ausführungsbeispiel an seiner Rückseite mit Verdrehanschlägen 9i versehen, die in der hinteren axialen Endposition des Dosiseinstellglieds 9 in Eingriff mit den Verdrehanschlägen 12i gelangen. In der hinteren axialen Endposition des Dosiseinstellglieds 9 wird durch das hintere Verdrehanschlagpaar 9i/12i nur diejenige rotatorische Dosierbewegung zugelassen, die eine translatorische Dosierbewegung des Dosiseinstellglieds 9 in die Vorschubrichtung bewirkt.

### Bezugszeichen:

- 1: Reservoirteil, Ampullenhalter
- 2: Reservoir, Ampulle
- 3: Mechanikhalter
- 3a: Erstes Verriegelungselement
- 3b: Fixiereinrichtung
- 3c: Ausschüttanschlag, Translationsanschlag
- 3d: Axialführung
- 3e: Wulst
- 3f: Federelement
- 3g: Rastnase
- 3h: Verdrehanschlag
- 4: Kolbenstange
- 4a: Verbindungsabschnitt
- 5: Gewindeabschnitt
- 6: Rückzugsperreinrichtung, Zahnreihe
- 7: Vorschubbremseinrichtung, Zahnreihe
- 8: Blockiereinrichtung
- 8a: Sperrelement
- 8b: Bremselement
- 8h: Verdrehanschlag
- 9: Dosiseinstellglied
- 9h: Verdrehanschlag
- 9i: Verdrehanschlag
- 10: Reservoirmodul
- 11: hinterer Gehäuseabschnitt
- 11d: Eingriffselement
- 11i: Verdrehanschlag
- 12: Dosier- und Betätigungselement
- 12i: Verdrehanschlag
- 13: Dosiermitnehmer
- 14: Verschluss
- 15a: Mitnehmer, Ringsteg
- 15b: Mitnehmer, Ringsteg
- 16: Rückstelleinrichtung
- 17: Zähl- und Anzeigeeinrichtung
- 18: -
- 19: -
- 20: Verriegelungsring
- 21: Zweites Verriegelungselement
- 22: Entriegelungsknopf
- 23: Sperrnocken
- 24: Rückstelleinrichtung
- 25: Verriegelungssperre
- 26: Mitnehmer, Steg
- 27: Axialausnehmung
- 28: Sperrzunge
- 29: Einrückausnehmung
- 30: Dosier- und Betätigungsmodul
- 31: Anschlagelement
- 32: Dosier- und Betätigungselement
- 33-37: -
- 38: Blockiereinrichtung
- 39: Dosiseinstellglied
- 39a: Dosiergewinde
- 39c: Ausschüttanschlag
- 39d: Axialführung
- 39g: Rastaufnahme, Axialführung
- 39h: Verdrehanschlag
- 39i: Verdrehanschlag

## Patentansprüche

1. Produktabgabegerät mit einem Reservoirmodul (10) und einem lösbar mit dem Reservoirmodul verbundenen Dosier- und Betätigungsmodul (30), das Reservoirmodul (10) umfassend:
a) einen vorderen Gehäuseabschnitt (1, 3) des Produktabgabegeräts, der ein Reservoir (2) für ein ausschüttbares Produkt, eine Blockiereinrichtung (8; 38) und eine erste Verbindungseinrichtung (3a, 3d) aufweist,
b) einen Kolben, der in dem Reservoir (2) in eine Vorschubrichtung auf einen Reservoirauslass zu verschiebbar aufgenommen ist, um Produkt auszuschütten,
c) und eine Kolbenstange (4), die eine Rückzugssperreinrichtung (6) aufweist, die mit der Blockiereinrichtung (8; 38) in einem Sperreingriff ist, der eine Bewegung der Kolbenstange (4) relativ zu dem vorderen Gehäuseabschnitt (1, 3) entgegen der Vorschubrichtung verhindert, das Dosier- und Betätigungsmodul (30) umfassend:
d) einen hinteren Gehäuseabschnitt (11) des Produktabgabegeräts, der eine zweite Verbindungseinrichtung (20,21, 11d) aufweist, die mit der ersten Verbindungseinrichtung (3a, 3d) die lösbare Verbindung zwischen dem Reservoirmodul (10) und dem Dosier- und Betätigungsmodul(30) bildet,
e) und eine Dosier- und Antriebsvorrichtung (12 ; 32), mit der relativ zu wenigstens einem der Gehäuseabschnitte (1, 3, 11) eine Dosierbewegung in einer Ausgangsstellung durch Drehung der Dosier- und Antriebsvorrichtung um die Längsachse (L) der Dosier und Antriebsvorrichtung (12; 32) und zwar zur Auswahl einer Produktdosis, und eine Ausschüttbewegung aus der Ausgangsstellung durch Axialverschiebung der Dosier- und Betätigungsvorrichtung (12; 32) zur Ausschüttung der gewählten Produktdosis ausführbar sind,
f) wobei das Produktabgabegerät ferner ein Dosiseinstellglied (9; 39) umfasst, das von der Dosier- und Antriebsvorrichtung (12; 32) bei der Ausschüttbewegung in die Vorschubrichtung bewegt wird und das mit der Kolbenstange (4) in solch einem Eingriff steht, dass es gemeinsam mit der Kolbenstange (4) in die Vorschubrichtung und durch die Dosierbewegung relativ zu der Kolbenstange (4) entgegen der Vorschubrichtung bewegt wird.

2. Produktabgabegerät nach dem vorhergehenden Anspruch, **dadurch gekennzeichnet, dass** die erste Verbindungseinrichtung (3a, 3d) und die zweite Verbindungseinrichtung (20, 21, 11d) eine axiale Linearführung (3d, 11d) bilden, um eine Relativdrehung zwischen dem vorderen Gehäuseabschnitt (1, 3) und dem hinteren Gehäuseabschnitt (11) zu verhindern, wenn der vordere Gehäuseabschnitt (1, 3) mit dem hinteren Gehäuseabschnitt (11) verbunden wird.

3. Produktabgabegerät nach einem der vorhergehenden Ansprüche, **dadurch gekennzeichnet, dass** die erste Verbindungseinrichtung (3a, 3d) ein erstes Verriegelungselement (3 a) und die zweite Verbindungseinrichtung (20, 21, 11d) ein zweites Verriegelungselement (21) umfasst und die Verriegelungselemente (3a, 21) in einem Verriegelungseingriff sind, um die Gehäuseabschnitte (1, 3, 11) axial aneinander zu fixieren.

4. Produktabgabegerät nach dem vorhergehenden Anspruch, **dadurch gekennzeichnet, dass** wenigstens eines (21) der Verriegelungselemente (3a, 21) mit einem (11) der Gehäuseabschnitte (1, 3, 11) gegen eine Elastizitätskraft radial bewegbar verbunden ist.

5. Produktabgabegerät nach einem der vorhergehenden Ansprüche, **dadurch gekennzeichnet, dass** das Dosiseinstellglied (9; 39) von einem (1, 3) der Gehäuseabschnitte (1, 3, 11) axial linear geführt wird.

6. Produktabgabegerät nach einem der vorhergehenden Ansprüche, **dadurch gekennzeichnet, dass** das Dosiseinstellglied (39) in vorgegebenen Drehwinkelpositionen mit einem (1, 3) der Gehäuseabschnitte (1, 3, 11) je in einem lösbaren Eingriff ist und vorzugsweise in dem Eingriff axial linear geführt wird, wobei der Eingriff vorzugsweise ein Rasteingriff ist.

7. Produktabgabegerät nach dem vorhergehenden Anspruch, **dadurch gekennzeichnet, dass** eine Rastnase (3g) und eine Rastaufnahme (39g), von denen die eine an dem Dosiseinstellglied (39) und die andere an dem einen (1, 3) der Gehäuseabschnitte (1, 3; 11) gebildet ist und die miteinander in dem Rasteingriff stehen, gegen eine rückstellende Elastizitätskraft aus dem Rasteingriff bewegbar sind.

8. Produktabgabegerät nach einem der vorhergehenden Ansprüche, **dadurch gekennzeichnet, dass** das Dosiseinstellglied (39) wenigstens einen Anschlag (39h; 39i) aufweist, der in einer Dosierendstellung des Dosiseinstellglieds (39) mit einem der Gehäuseabschnitte (1, 3, 11) in einem Sperreingriff steht, der eine Bewegung des Dosiseinstellglieds (39) verhindert, die eine Reaktionsbewegung der Kolbenstange (4) entgegen der Vorschubrichtung bewirken könnte.

9. Produktabgabegerät nach einem der vorhergehenden Ansprüche, **dadurch gekennzeichnet, dass** der vordere Gehäuseabschnitt (1, 3) einen Ausschüttanschlag (3c) für das Dosiseinstellglied (9; 39) bildet, der die Ausschüttbewegung in die Vorschubrichtung begrenzt.

10. Produktabgabegerät nach einem der vorhergehenden Ansprüche, **dadurch gekennzeichnet, dass** die Dosier- und Antriebsvorrichtung (32) und das Dosiseinstellglied (39) miteinander in einem Eingriff sind, der eine Mitnahme des Dosiseinstellglieds (39) bei der Dosierbewegung bewirkt und eine Bewegung der Dosier- und Antriebsvorrichtung (32) in und entgegen die Vorschubrichtung relativ zu dem Dosiseinstellglied (39) zulässt, wobei das Dosiseinstellglied (39) einen Anschlag (39c) für die Dosier- und Antriebsvorrichtung (32) bildet, gegen den die Dosier- und Antriebsvorrichtung (32) bei ihrer Ausschüttbewegung drückt, um das Dosiseinstellglied (39) in die Vorschubrichtung zu bewegen.

11. Produktabgabegerät nach einem der Ansprüche 1 bis 9, **dadurch gekennzeichnet, dass** die Dosier- und Antriebsvorrichtung (12) und die Kolbenstange (4) miteinander in einem Eingriff sind, der eine Mitnahme der Kolbenstange (4) bei der Dosierbewegung bewirkt, aber eine Ausschüttbewegung der Dosier- und Antriebsvorrichtung (12) relativ zu der Kolbenstange (4) zulässt.

12. Produktabgabegerät nach einem der vorhergehenden Ansprüche, **dadurch gekennzeichnet, dass** die Dosierbewegung eine Drehbewegung um eine Längsachse (L) der Kolbenstange (4) ist.

13. Produktabgabegerät nach dem vorhergehenden Anspruch, **dadurch gekennzeichnet, dass** die Kolbenstange (4) und das Dosiseinstellglied (9; 39) miteinander in einem Gewindeeingriff sind.

14. Produktabgabegerät nach einem der vorhergehenden Ansprüche, **dadurch gekennzeichnet, dass** eine Kanüle von höchstens 30 Gauge, vorzugsweise eine 31 oder 32 Gauge Kanüle, oder eine Kanüle, die eine nicht in ISO-9626 spezifizierte Kombination von Außendurchmesser und Innendurchmesser aufweist, mit einem Außendurchmesser von höchstens 320 µm und einer möglichst dünnen Wandstärke eine Injektionskanüle des Produktabgabegeräts bildet.

15. Reservoirmodul für ein Produktabgabegerät, wobei das Reservoirmodul lösbar mit dem Produktabgabegerät verbindbar ist, und wobei das Reservoirmodul (10) umfasst:
a) einen vorderen Gehäuseabschnitt (1, 3) des Produktabgabegeräts, der ein Reservoir (2) für ein ausschüttbares Produkt, eine Blockiereinrichtung (8; 38) und eine Verbindungseinrichtung (3a, 3d) zur Herstellung einer Verbindung mit einem Dosier- und Betätigungsmodul (30) des Produktabgabegeräts aufweist,
b) einen Kolben, der in dem Reservoir (2) in eine Vorschubrichtung auf einen Resrvoirauslass zu bewegbar aufgenommen ist, um Produkt auszuschütten,
c) ein Dosiseinstellglied (9; 39), das von dem vorderen Gehäuseabschnitt (1, 3) bewegbar aufgenommen wird, um eine Dosierbewegung in einer Ausgangsstellung durch Drehung der Dosier- und Antriebsvorrichtung um die Längsachse (L) der Dosier- und Antriebsvorrichtung (12; 32) vorgenommen werden, und zwar zur Auswahl einer Produktdosis und eine Ausschüttbewegung aus der Ausgangsstellung durch Axialverschiebung der Dosier-und Betätigungsvorrichtung (12; 32) zur Ausschüttung der ausgewählten Produktdosis auszuführen,
d) und eine Kolbenstange (4), die mit dem Dosiseinstellglied (9 ; 39) verbunden ist und von dem vorderen Gehäuseabschnitt (1, 3) so gehalten wird, dass eine Bewegung der Kolbenstange (4) in Vorschubrichtung nicht durch die Dosierbewegung bewirkt wird,
e) wobei die Kolbenstange (4) eine Rückzugssperreinrichtung (6) aufweist, die mit der Blockiereinrichtung (8 ; 38) in einem Sperreingriff ist, der eine Bewegung der Kolbenstange (4) relativ zu dem vorderen Gehäuseabschnitt (1, 3) entgegen der Vorschubrichtung verhindert.

16. Reservoirmodul nach dem vorhergehenden Anspruch, **dadurch gekennzeichnet, dass** die Blockiereinrichtung (8; 38) und die Kolbenstange (4) in einem Sicherheitseingriff sind, der verhindert, dass die Kolbenstange (4) in eine Stellung rückführbar ist, die sie vor der Ausführung einer in die Vorschubrichtung gerichteten Bewegung eingenommen hat, und dass der Sicherheitseingriff nicht lösbar ist.

17. Reservoirmodul nach dem vorhergehenden Anspruch, **dadurch gekennzeichnet, dass** der Sperreingriff zwischen der Blockiereinrichtung (8; 38) und der Rückzugsperreinrichtung (6) den Sicherheitseingriff bildet.

18. Reservoirmodul nach einem der vorhergehenden Ansprüche, **dadurch gekennzeichnet, dass** der vordere Gehäuseabschnitt (1, 3) ein hülsenförmiges Reservoirteil (1) mit dem Reservoir (2) und einen hülsenförmigen Mechanikhalter (3) umfasst, die separat gefertigt und so miteinander verbunden sind, dass ein Benutzer die Verbindung zerstörungsfrei nicht lösen kann, wobei der Mechanikhalter (3) die Kolbenstange (4) hält.

19. Reservoirmodul nach dem vorhergehenden Anspruch, **dadurch gekennzeichnet, dass** der Mechanikhalter (3) einen Ausschüttanschlag (3c) für das Dosiseinstellglied (9; 39) bildet, um die Ausschüttbewegung zu begrenzen, wobei das Dosiseinstellglied (9; 39) durch die Dosierbewegung entgegen der Vorschubrichtung von dem Ausschüttanschlag (3c) wegbewegt wird.

20. Reservoirmodul nach einem der vorhergehenden Ansprüche, **dadurch gekennzeichnet, dass** der vordere Gehäuseabschnitt (1, 3) eine Gleitführung für das Dosiseinstellglied (9; 39) bildet.

21. Reservoirmodul nach einem der vorhergehenden Ansprüche, **dadurch gekennzeichnet, dass** der vordere Gehäuseabschnitt (1, 3) das Dosiseinstellglied (9; 39) axial linearführt.

22. Reservoirmodul nach dem vorhergehenden Anspruch, **dadurch gekennzeichnet, dass** das Dosiseinstellglied (39) in vorgegebenen Drehwinkelpositionen mit dem vorderen Gehäuseabschnitt (1, 3) je in einem lösbaren Eingriff ist und vorzugsweise in dem Eingriff axial lineargeführt wird, wobei der Eingriff vorzugsweise ein Rasteingriff ist.

23. Reservoirmodul nach dem vorhergehenden Anspruch, **dadurch gekennzeichnet, dass** eine Rastnase (3g) und eine Rastaufnahme (39g), von denen die eine an dem vorderen Gehäuseabschnitt (1, 3) und die andere an dem Dosiseinstellglied (39) ausgebildet ist, miteinander in dem Rasteingriff sind und gegen eine rückstellende Elastizitätskraft aus dem Rasteingriff bewegbar sind.

24. Reservoirmodul nach einem der vorhergehenden Ansprüche, **dadurch gekennzeichnet, dass** an dem vorderen Gehäuseabschnitt (1, 3) eine Axialführung (3d) mit wenigstens einer geraden axialen Führungsfläche geformt ist, um im Zusammenwirken mit einem an dem hinteren Gehäuseabschnitt (11) geformten Eingriffsglied eine Linearführung zwischen dem vorderen Gehäuseabschnitt (1,3) und dem hinteren Gehäuseabschnitt (11) zu bilden.

25. Reservoirmodul nach einem der vorhergehenden Anspruch, **dadurch gekennzeichnet, dass** das Dosiseinstellglied (39) und der vordere Gehäuseabschnitt (1, 3) je wenigstens einen Anschlag (3h, 39h) aufweisen, die in einer vorderen Dosierendstellung des Dosiseinstellglieds (39) in Eingriff sind, um eine Bewegung des Dosiseinstellglieds (39) zu verhindern, die eine Reaktionsbewegung der Kolbenstange (4) entgegen der Vorschubrichtung bewirken könnte.

26. Reservoirmodul nach einem der vorhergehenden Ansprüche, **dadurch gekennzeichnet, dass** die Blockiereinrichtung (38) mit dem vorderen Gehäuseabschnitt (1, 3) unbeweglich verbunden ist und eine Verdrehsicherung bildet, die eine Drehbewegung der Kolbenstange (4) relativ zu dem vorderen Gehäuseabschnitt (1,3) verhindert.

27. Reservoirmodul nach einem der Ansprüche 15 bis 25, **dadurch gekennzeichnet, dass** die Blockiereinrichtung (8) von dem vorderen Gehäuseabschnitt (1, 3) um die Längsachse der Kolbenstange (4) drehbar und entgegen der Vorschubrichtung nicht bewegbar gelagert wird.

28. Reservoirmodul nach einem der vorhergehenden Ansprüche, **dadurch gekennzeichnet, dass** die Kolbenstange (4) eine Vorschubbremseinrichtung (7) aufweist und die Blockiereinrichtung (8) des vorderen Gehäuseabschnitts mit der Vorschubbremseinrichtung (7) in einem Bremseingriff ist, der die Bewegung der Kolbenstange (4) in die Vorschubrichtung erschwert.

29. Reservoirmodul nach einem der vorhergehenden Ansprüche, **dadurch gekennzeichnet, dass** die Rückzugssperreinrichtung (6) der Kolbenstange (4) von wenigstens einer Sägezahnreihe gebildet wird.

30. Reservoirmodul nach einem der vorhergehenden Ansprüche, **dadurch gekennzeichnet, dass** das Reservoirmodul (10) ein Verbrauchsmodul ist, dass einer Entleerung des Reservoirs (2) für einen Austausch im Ganzen vorgesehen ist.

## Claims

1. A product dispensing apparatus, comprising a reservoir module (10) and a dosing and activating module (30) detachably connected to the reservoir module, said reservoir module (10) comprising:
a) a front casing section (1, 3) of the product dispensing apparatus, which comprises a reservoir (2) for a product which can be delivered, a blocking means (8; 38) and a first connecting means (3a, 3d);
b) a piston which is accommodated in the reservoir (2) such that it can be shifted in an advancing direction towards a reservoir outlet, in order to deliver product;
c) and a piston rod (4) which comprises a returning blocking means (6) which is in blocking engagement with the blocking means (8; 38), the blocking engagement preventing the piston rod (4) from moving counter to the advancing direction, relative to the front casing section (1, 3), said dosing and activating module (30) comprising:
d) a rear casing section (11) of the product dispensing apparatus, which comprises a second connecting means (20, 21, 11d) which together with the first connecting means (3a, 3d) forms the detachable connection between the reservoir module (10) and the dosing and activating module (30);
e) and a dosing and drive device (12; 32), using which a dosing movement relative to at least one of the casing sections (1, 3, 11) for selecting a product dosage can be performed in an initial position by rotating the dosing and drive device about the longitudinal axis (L) of the dosing and drive device (12; 32), and a delivery movement relative to at least one of the casing sections (1, 3, 11) for delivering the selected product dosage can be performed from the initial position by axially shifting the dosing and activating device (12; 32);
f) wherein the product dispensing apparatus further comprises a dosage setting member (9; 39) which is moved in the advancing direction by the dosing and drive device (12; 32) during the delivery movement and which engages with the piston rod (4) such that it is moved jointly with the piston rod (4) in the advancing direction and is moved relative to the piston rod (4) counter to the advancing direction by the dosing movement.

2. The product dispensing apparatus according to the preceding claim, **characterised in that** the first connecting means (3a, 3d) and the second connecting means (20, 21, 11d) form an axial linear guide (3d, 11d), to prevent the front casing section (1, 3) and the rear casing section (11) from rotating relative to each other when the front casing section (1, 3) is connected to the rear casing section (11).

3. The product dispensing apparatus according to any one of the preceding claims, **characterised in that** the first connecting means (3a, 3d) comprises a first latching element (3a) and the second connecting means (20, 21, 11d) comprises a second latching element (21) and the latching elements (3a, 21) are in latching engagement, in order to axially fix the casing sections (1, 3, 11) onto each other.

4. The product dispensing apparatus according to the preceding claim, **characterised in that** at least one (21) of the latching elements (3 a, 21) is connected, radially movable, to one (11) of the casing sections (1, 3, 11), against an elasticity force.

5. The product dispensing apparatus according to any one of the preceding claims, **characterised in that** the dosage setting member (9; 39) is axially and linearly guided by one (1, 3) of the casing sections (1, 3, 11).

6. The product dispensing apparatus according to any one of the preceding claims, **characterised in that** the dosage setting member (39) engages, detachably in each case, with one (1, 3) of the casing sections (1, 3, 11) in pre-set rotational angular positions and when engaged is preferably axially and linearly guided, the engagement preferably being a locking engagement.

7. The product dispensing apparatus according to the preceding claim, **characterised in that** a locking projection (3g) and a locking recess (39g), one of which is formed on the dosage setting member (39) and the other on one (1, 3) of the casing sections (1, 3, 11) and which are in locking engagement with each other, can be moved out of locking engagement against a restoring elasticity force.

8. The product dispensing apparatus according to any one of the preceding claims, **characterised in that** the dosage setting member (39) comprises at least one stopper (39h; 39i) which in the dosing end position of the dosage setting member (39) is in blocking engagement with one of the casing sections (1, 3, 11), the blocking engagement preventing a movement of the dosage setting member (39) which could cause a response movement by the piston rod (4) counter to the advancing direction.

9. The product dispensing apparatus according to any one of the preceding claims, **characterised in that** the front casing section (1, 3) forms a delivery stopper (3c) for the dosage setting member (9; 39), the delivery stopper limiting the delivery movement in the advancing direction.

10. The product dispensing apparatus according to any one of the preceding claims, **characterised in that** the dosing and drive device (32) and the dosage setting member (39) engage with each other, wherein the engagement slaves the dosage setting member (39) during the dosing movement and allows the dosing and drive device (32) to move in and counter to the advancing direction relative to the dosage setting member (39), wherein the dosage setting member (39) forms a stopper (39c) for the dosing and drive device (32) against which the dosing and drive device (32) pushes during its delivery movement, in order to move the dosage setting member (39) in the advancing direction.

11. The product dispensing apparatus according to any one of claims 1 to 9, **characterised in that** the dosing and drive device (12) and the piston rod (4) engage with each other, wherein the engagement slaves the piston rod (4) during the dosing movement but allows a delivery movement of the dosing and drive device (12) relative to the piston rod (4).

12. The product dispensing apparatus according to any one of the preceding claims, **characterised in that** the dosing movement is a rotational movement about a longitudinal axis (L) of the piston rod (4).

13. The product dispensing apparatus according to the preceding claim, **characterised in that** the piston rod (4) and the dosage setting member (9; 39) are in threaded engagement with each other.

14. The product dispensing apparatus according to any one of the preceding claims, **characterised in that** a cannula of at most 30 gauge, preferably a 31 or 32 gauge cannula, or a cannula exhibiting a combination of outer and inner diameter not specified in ISO 9626, having an outer diameter of 320 µm at most and as thin a wall thickness as possible, forms an injection cannula of the product dispensing apparatus.

15. A reservoir module for a product dispensing apparatus, wherein the reservoir module can be detachably connected to the product dispensing apparatus, said reservoir module (10) comprising:
a) a front casing section (1, 3) of the product dispensing apparatus, which comprises a reservoir (2) for a product which can be delivered, a blocking means (8; 38) and a connecting means (3a, 3d) for establishing a connection to a dosing and activating module (30) of the product dispensing apparatus;
b) a piston which is accommodated in the reservoir (2) such that it can be moved in an advancing direction towards a reservoir outlet, in order to deliver product;
c) a dosage setting member (9; 39) which is accommodated by the front casing section (1, 3) such that it can be moved, in order to perform a dosing movement in an initial position by rotating the dosing and drive device about the longitudinal axis (L) of the dosing and drive device (12; 32), in order to select a product dosage, and to perform a delivery movement from the initial position by axially shifting the dosing and activating device (12; 32), in order to deliver the selected product dosage;
d) and a piston rod (4) which is connected to the dosage setting member (9; 39) and held by the front casing section (1, 3) such that the dosing movement does not cause the piston rod (4) to move in the advancing direction;
e) wherein the piston rod (4) comprises a returning blocking means (6) which is in blocking engagement with the blocking means (8; 38), the blocking engagement preventing the piston rod (4) from moving counter to the advancing direction, relative to the front casing section (1, 3).

16. The reservoir module according to the preceding claim, **characterised in that** the blocking means (8; 38) and the piston rod (4) are in securing engagement, the securing engagement preventing the piston rod (4) from being returned to a position which it assumed before performing a movement in the advancing direction, and **in that** the securing engagement is not releasable.

17. The reservoir module according to the preceding claim, **characterised in that** the blocking engagement between the blocking means (8; 38) and the returning blocking means (6) forms the securing engagement.

18. The reservoir module according to any one of the preceding claims, **characterised in that** the front casing section (1, 3) comprises a sleeve-shaped reservoir part (1) comprising the reservoir (2) and a sleeve-shaped mechanism holder (3), which are produced separately and connected to each other such that a user cannot release the connection without destroying it, wherein the mechanism holder (3) holds the piston rod (4).

19. The reservoir module according to the preceding claim, **characterised in that** the mechanism holder (3) forms a delivery stopper (3c) for the dosage setting member (9; 39), in order to limit the delivery movement, wherein the dosing movement moves the dosage setting member (9; 39) counter to the advancing direction, away from the delivery stopper (3c).

20. The reservoir module according to any one of the preceding claims, **characterised in that** the front casing section (1, 3) forms a sliding guide for the dosage setting member (9; 39).

21. The reservoir module according to any one of the preceding claims, **characterised in that** the front casing section (1, 3) axially and linearly guides the dosage setting member (9; 39).

22. The reservoir module according to the preceding claim, **characterised in that** the dosage setting member (39) engages, detachably in each case, with the front casing section (1, 3) in pre-set rotational angular positions and when engaged is preferably axially and linearly guided, the engagement preferably being a locking engagement.

23. The reservoir module according to the preceding claim, **characterised in that** a locking projection (3g) and a locking recess (39g), one of which is formed on the front casing section (1, 3) and the other on the dosage setting member (39), are in locking engagement with each other and can be moved out of locking engagement against a restoring elasticity force.

24. The reservoir module according to any one of the preceding claims, **characterised in that** an axial guide (3d) comprising at least one straight, axial guide area is formed on the front casing section (1, 3), in order - in co-operation with an engagement member formed on the rear casing section (11) - to form a linear guide between the front casing section (1, 3) and the rear casing section (11).

25. The reservoir module according to any one of the preceding claims, **characterised in that** the dosage setting member (39) and the front casing section (1, 3) each comprise at least one stopper (3h, 39h) which engage in a front dosing end position of the dosage setting member (39), in order to prevent a movement of the dosage setting member (39) which could cause a response movement by the piston rod (4) counter to the advancing direction.

26. The reservoir module according to any one of the preceding claims, **characterised in that** the blocking means (38) is connected immovably to the front casing section (1, 3) and forms a rotational block which prevents the piston rod (4) from rotating relative to the front casing section (1, 3).

27. The reservoir module according to any one of claims 15 to 25, **characterised in that** the blocking means (8) is mounted by the front casing section (1, 3) such that it can rotate about the longitudinal axis of the piston rod (4) and cannot be moved counter to the advancing direction.

28. The reservoir module according to any one of the preceding claims, **characterised in that** the piston rod (4) comprises an advancing braking means (7), and the blocking means (8) of the front casing section is in braking engagement with the advancing braking means (7), the braking engagement making it more difficult for the piston rod (4) to move in the advancing direction.

29. The reservoir module according to any one of the preceding claims, **characterised in that** the returning blocking means (6) of the piston rod (4) is formed by at least one row of serrated teeth.

30. The reservoir module according to any one of the preceding claims, **characterised in that** the reservoir module (10) is a disposable module which is provided to be exchanged in its entirety once the reservoir (2) has been emptied.

## Revendications

1. Appareil d'administration de produit, comportant un module de réservoir (10) et un module de dosage et d'actionnement (30) relié de façon détachable au module de réservoir, le module de réservoir (10) comprenant :
a) un tronçon de boîtier antérieur (1, 3) de l'appareil d'administration de produit, qui présente un réservoir (2) pour un produit à déverser, un dispositif de blocage (8 ; 38) et un premier dispositif de liaison (3a, 3d),
b) un piston, qui est logé dans le réservoir, déplaçable vers une sortie de réservoir dans une direction de défilement afin de déverser le produit,
c) et une tige de piston (4) qui comprend un dispositif de blocage anti-retour (6) qui est en engagement d'arrêt avec le dispositif de blocage (8 ; 38), engagement qui interdit un mouvement de la tige de piston (4) par rapport au tronçon de boîtier antérieur (1, 3) en sens opposé à la direction de défilement, le module de dosage et l'actionnement (30) comprenant :
d) un tronçon de boîtier postérieur (11) de l'appareil d'administration de produit, qui présente un second dispositif de liaison (20, 21, 11d) qui établit conjointement avec le premier dispositif de liaison (3a, 3d) la liaison détachable entre le module de réservoir (10) et le module de dosage et d'actionnement (30),
e) un dispositif de dosage et d'entraînement (12 ; 32) au moyen duquel peut s'effectuer un mouvement de dosage par rapport à l'un au moins des tronçons de boîtier (1, 3, 11) dans une position de départ par rotation du dispositif de dosage et d'entraînement autour de l'axe longitudinal (L) du dispositif de dosage et d'entraînement (12 ; 32), et ceci pour choisir une dose de produit, ainsi qu'un mouvement de déversement à partir de la positon de départ par translation axiale du dispositif de dosage et d'actionnement (12 ; 32) en vue de déverser la dose de produit choisie,
f) l'appareil d'administration de produit comprenant en outre un organe de réglage de dose (9 ; 39) qui est déplacé dans la direction de défilement par le dispositif de dosage et d'entraînement (12 ; 32) lors du mouvement de déversement et qui est en engagement avec la tige de piston (4) de manière à être déplacé conjointement avec la tige de piston (4) dans la direction de défilement et par le mouvement de dosage par rapport à la tige de piston (4) en sens opposé à la direction de défilement.

2. Appareil d'administration de produit selon la revendication précédente, **caractérisé en ce que** le premier dispositif de liaison (3a, 3d) et le second dispositif de liaison (20, 21, 11d) forment un guidage linéaire axial (3d, 11d), afin d'empêcher une rotation relative entre le tronçon de boîtier antérieur (1, 3) et le tronçon de boîtier postérieur (11) lorsque le tronçon de boîtier antérieur (1, 3) est relié au tronçon de boîtier postérieur (11).

3. Appareil d'administration de produit selon l'une des revendications précédentes, **caractérisé en ce que** le premier dispositif de liaison (3a, 3d) comprend un premier élément de verrouillage (3a) et le second dispositif de liaison (20, 21, 11d) comprend un second élément de verrouillage (21), et les éléments de verrouillage (3a, 21) sont en engagement de verrouillage pour fixer axialement les tronçons de boîtier (1,3, 11) l'un à l'autre.

4. Appareil d'administration de produit selon la revendication précédente, **caractérisé en ce que** l'un au moins (21) des éléments de verrouillage (3a, 21) est relié à l'un (11) des tronçons de boîtier (1, 3, 11) déplaçable radialement à l'encontre d'une force élastique.

5. Appareil d'administration de produit selon l'une des revendications précédentes, **caractérisé en ce que** l'organe de réglage de dose (9 ; 39) est guidé linéairement axialement par l'un (1, 3) des tronçons de boîtier (1,3,11).

6. Appareil d'administration de produit selon l'une des revendications précédentes, **caractérisé en ce que** l'organe de réglage de dose (39) est en engagement respectif détachable avec l'un (1, 3) des tronçons de boîtier (1, 3, 11) dans des positions angulaires de rotation prédéterminées et, dans cet engagement, il est de préférence guidé linéairement axialement, l'engagement étant de préférence un engagement par enclenchement.

7. Appareil d'administration de produit selon la revendication précédente, **caractérisé en ce qu'**un bec d'enclenchement (3g) et un logement d'enclenchement (39g) dont l'un est formé sur l'organe de réglage de dose (39) et l'autre est formé sur l'un (1, 3) des tronçons de boîtier (1, 3 ; 11) et qui sont en engagement par enclenchement l'un avec l'autre sont mobiles hors de l'engagement par enclenchement à l'encontre d'une force élastique de rappel.

8. Appareil d'administration de produit selon l'une des revendications précédentes, **caractérisé en ce que** l'organe de réglage de dose (39) comprend au moins une butée (39h ; 39i) qui, dans une position de dosage terminale de l'organe de réglage de dose (39), est en engagement d'arrêt avec l'un des tronçons de boîtier (1, 3, 11), qui empêche un mouvement de l'organe de réglage de dose (39) qui pourrait provoquer un mouvement de réaction de la tige de piston (4) en sens opposé à la direction de défilement.

9. Appareil d'administration de produit selon l'une des revendications précédentes, **caractérisé en ce que** le tronçon de boîtier antérieur (1, 3) forme une butée de déversement (3c) pour l'organe de réglage de dose (9 ; 39) qui limite le mouvement de déversement dans la direction de défilement.

10. Appareil d'administration de produit selon l'une des revendications précédentes, **caractérisé en ce que** le dispositif de dosage et d'entraînement (32) et l'organe de réglage de dose (39) sont en engagement l'un avec l'autre, qui provoque un entraînement de l'organe de réglage de dose (39) lors du mouvement de dosage et qui autorise un mouvement du dispositif de dosage et d'entraînement (32) dans et en sens opposé à la direction de défilement par rapport à l'organe de réglage de dose (39), l'organe de réglage de dose (39) formant une butée (39c) pour le dispositif d'entraînement et de dosage (32) contre laquelle pousse le dispositif de dosage et d'entraînement (32) lors de son mouvement de déversement, afin de déplacer l'organe de réglage de dose (39) dans la direction de défilement.

11. Appareil d'administration de produit selon l'une des revendications 1 à 9, **caractérisé en ce que** le dispositif de dosage et d'entraînement (12) et la tige de piston (4) sont en un engagement l'un avec l'autre qui provoque un entraînement de la tige de piston (4) lors du mouvement de dosage, mais qui autorise un mouvement de déversement du dispositif de dosage et d'entraînement (12) par rapport à la tige de piston (4).

12. Appareil d'administration de produit selon l'une des revendications précédentes, **caractérisé en ce que** le mouvement de dosage est un mouvement de rotation autour d'un axe longitudinal (L) de la tige de piston (4).

13. Appareil d'administration de produit selon la revendication précédente, **caractérisé en ce que** la tige de piston (4) et l'organe de réglage de dose (9 ; 39) sont en engagement à pas de vis l'un avec l'autre.

14. Appareil d'administration de produit selon l'une des revendications précédentes, **caractérisé en ce qu'**une canule au calibre maximum de 30, de préférence une canule au calibre 31 ou 32, ou bien une canule qui présente une combinaison de diamètre extérieur et de diamètre intérieur hors des spécifications de la norme ISO-9626, d'un diamètre extérieur de 320 µm au maximum et d'une épaisseur de paroi aussi mince que possible, forme une canule d'injection de l'appareil d'administration de produit.

15. Module de réservoir pour un appareil d'administration de produit, le module de réservoir étant susceptible d'être relié de façon détachable à l'appareil d'administration de produit, le module de réservoir (10) comprenant :
a) un tronçon de boîtier antérieur (1, 3) de l'appareil d'administration de produit, qui présente un réservoir (2) pour un produit à déverser, un dispositif de blocage (8 ; 38) et un dispositif de liaison (3a, 3d) pour établir une liaison avec un module de dosage et d'actionnement (30) de l'appareil d'administration de produit,
b) un piston, qui est logé dans le réservoir (2), déplaçable vers une sortie de réservoir dans une direction de défilement afin de déverser le produit,
c) un organe de réglage de dose (9 ; 39) qui est reçu avec possibilité de déplacement par le tronçon de boîtier antérieur (1, 3) pour réaliser un mouvement de dosage dans une position de départ par rotation du dispositif de dosage et d'entraînement autour de l'axe longitudinal (L) du dispositif de dosage et d'entraînement (12 ; 32), et ceci pour choisir une dose de produit et un mouvement de déversement à partir de la position de départ par translation axiale du dispositif de dosage et d'actionnement (12 ; 32) en vue de déverser la dose de produit choisie,
d) et une tige de piston (4) qui est reliée à l'organe de réglage de dose (9 ; 39) et qui est retenue par le tronçon de boîtier antérieur (1, 3) de telle sorte qu'un mouvement de la tige de piston (4) dans la direction de défilement n'est pas provoqué par le mouvement de dosage,
e) la tige de piston (4) comprenant un dispositif de blocage anti-retour (6) qui est en engagement d'arrêt avec le dispositif de blocage (8 ; 38), engagement qui interdit un mouvement de la tige de piston (4) par rapport au tronçon de boîtier antérieur (1, 3) en sens opposé à la direction de défilement.

16. Module de réservoir selon la revendication précédente, **caractérisé en ce que** le dispositif de blocage (8 ; 38) et la tige de piston (4) sont en engagement de sécurité qui empêche que la tige de piston (4) puisse retourner dans une position qu'elle a occupée avant l'exécution d'un mouvement dirigé dans la direction de défilement, et **en ce que** l'engagement de sécurité n'est pas détachable.

17. Module de réservoir selon la revendication précédente, **caractérisé en ce que** l'engagement d'arrêt entre le dispositif de blocage (8 ; 38) et le dispositif de blocage anti-retour (6) forme l'engagement de sécurité.

18. Module de réservoir selon l'une des revendications 15 à 17, **caractérisé en ce que** le tronçon de boîtier antérieur (1, 3) comprend une partie réservoir (1) en forme de douille avec le réservoir (2) et avec un porte-mécanisme en forme de douille (3) qui sont fabriqués séparément l'un de l'autre et reliés l'un à l'autre de telle sorte qu'un utilisateur ne peut pas défaire la liaison sans destruction, le porte-mécanisme (3) retenant la tige de piston (4).

19. Module de réservoir selon la revendication précédente, **caractérisé en ce que** le porte-mécanisme (3) forme une butée de déversement (3c) pour l'organe de réglage de dose (9 ; 39) pour limiter le mouvement de déversement, l'organe de réglage de dose (9 ; 39) étant déplacé en éloignement de la butée de déversement (3c) par le mouvement de dosage en sens opposé à la direction de défilement.

20. Module de réservoir selon l'une des revendications 15 à 19, **caractérisé en ce que** le tronçon de boîtier antérieur (1, 3) forme un guidage de coulissement pour l'organe de réglage de dose (9 ; 39).

21. Module de réservoir selon l'une des revendications 15 à 20, **caractérisé en ce que** le tronçon de boîtier antérieur (1, 3) guide linéairement axialement l'organe de réglage de dose (9; 39).

22. Module de réservoir selon la revendication précédente, **caractérisé en ce que** l'organe de réglage de dose (39) est en engagement respectif détachable avec le tronçon de boîtier antérieur (1, 3) dans des positions angulaires de rotation prédéterminées et, dans cet engagement, il est de préférence guidé linéairement axialement, l'engagement étant de préférence un engagement par enclenchement.

23. Module de réservoir selon la revendication précédente, **caractérisé en ce qu'**un bec d'enclenchement (3g) et un logement d'enclenchement (39g) dont l'un est formé sur le tronçon de boîtier antérieur (1, 3) et l'autre est formé sur l'organe de réglage de dose (39) sont en engagement par enclenchement l'un avec l'autre et mobiles hors de l'engagement par enclenchement à l'encontre d'une force élastique de rappel.

24. Module de réservoir selon l'une des revendications 15 à 23, **caractérisé en ce que** sur le tronçon de boîtier antérieur (1, 3) est formé un guidage axial (3d) avec au moins une surface de guidage axiale rectiligne, afin de former, en coopération avec un organe d'engagement formé sur le tronçon de boîtier postérieur (11), un guidage linéaire entre le tronçon de boîtier antérieur (1, 3) et le tronçon de boîtier postérieur (11).

25. Module de réservoir selon l'une des revendications 15 à 24, **caractérisé en ce que** l'organe de réglage de dose (39) et le tronçon de boîtier antérieur (1, 3) présentent chacun au moins une butée (3h, 39h) qui sont en engagement dans une position de dosage terminale de l'organe de réglage de dose (39), afin d'empêcher un mouvement de l'organe de réglage de dose (39) qui pourrait provoquer un mouvement de réaction de la tige de piston (4) en sens opposé à la direction de défilement.

26. Module de réservoir selon l'une des revendications 15 à 25, **caractérisé en ce que** le dispositif de blocage (38) est relié de façon immobile au tronçon de boîtier antérieur (1, 3) et forme un blocage anti-rotation qui empêche un mouvement de rotation de la tige de piston (4) par rapport au tronçon de boîtier antérieur (1, 3).

27. Module de réservoir selon l'une des revendications 15 à 26, **caractérisé en ce que** le dispositif de blocage (8) du tronçon de boîtier antérieur (1, 3) est monté de façon mobile en rotation autour de l'axe longitudinal de la tige de piston (4) et de façon immobile en sens opposé à la direction de défilement.

28. Module de réservoir selon l'une des revendications 15 à 27, **caractérisé en ce que** la tige de piston (4) présente un dispositif de freinage de défilement (7) et le dispositif de blocage (8) du tronçon de boîtier antérieur est en engagement de freinage avec le dispositif de freinage de défilement (7), qui rend difficile le mouvement de la tige de piston (4) dans la direction de défilement.

29. Module de réservoir selon l'une des revendications 15 à 28, **caractérisé en ce que** le dispositif de blocage anti-retour (6) de la tige de piston (4) est formé par au moins une rangée de dents de scie.

30. Module de réservoir selon l'une des revendications 15 à 29, **caractérisé en ce que** le module de réservoir (10) est un module consommable qui, après un vidage du réservoir (2), est prévu pour un remplacement global.
